# EUROPEAN PATENT SPECIFICATION

(11) **EP 3 735 319 B1**
(45) Date of publication and mention of the grant of the patent: **22.05.2024**
(21) Application number: 18700047.6
(22) Date of filing: 03.01.2018
(51) Int. Cl.: B01L 3/00, G01N 33/543, C12Q 1/6816, C12Q 1/6825

(54) **SINGLE-CHANNEL MULTIANALYTE BIOSENSOR**
EINKANAL-MULTIANALYTBIOSENSOR
BIOCAPTEUR D'ANALYTES MULTIPLES À CANAL UNIQUE

(43) Date of publication of application: 11.11.2020
(73) Proprietor: Albert-Ludwigs-Universität Freiburg, 79098 Freiburg (DE)
(72) Inventor: DINCER, Can, 79115 Freiburg (DE); KLING, André, 79104 Freiburg (DE); URBAN, Gerald A., 79104 Freiburg (DE); BRUCH, Richard, 79114 Freiburg (DE)
(74) Representative: Hertin und Partner Rechts- und Patentanwälte PartG mbB
(86) International application number: PCT/EP2018/050107
(87) International publication number: WO 2019/134741

(56) References cited:
- EP-A2- 2 950 096
- WO-A1-2014/075016
- ANDRÉ KLING ET AL: "Multianalyte Antibiotic Detection on an Electrochemical Microfluidic Platform", ANALYTICAL CHEMISTRY, vol. 88, no. 20, 1 August 2016 (2016-08-01), pages 10036-10043, XP055505295, US ISSN: 0003-2700, DOI: 10.1021/acs.analchem.6b02294
- L. ARMBRECHT ET AL: "Self-assembled magnetic bead chains for sensitivity enhancement of microfluidic electrochemical biosensor platforms", LAB ON A CHIP, vol. 15, no. 22, 1 January 2015 (2015-01-01), pages 4314-4321, XP055505392, ISSN: 1473-0197, DOI: 10.1039/C5LC00796H
- HORAK JOSEF ET AL: "Sensitive, rapid and quantitative detection of substance P in serum samples using an integrated microfluidic immunochip", BIOSENSORS & BIOELECTRONICS, vol. 58, August 2014 (2014-08), pages 186-192, XP002784553,
- C. DINCER ET AL: "Designed miniaturization of microfluidic biosensor platforms using the stop-flow technique", THE ANALYST, vol. 141, no. 21, 1 January 2016 (2016-01-01), pages 6073-6079, XP055505300, ISSN: 0003-2654, DOI: 10.1039/C6AN01330A
- NAOKI YANAGISAWA ET AL: "Multiplex ELISA in a single microfluidic channel", ANALYTICAL AND BIOANALYTICAL CHEMISTRY, SPRINGER, BERLIN, DE, vol. 401, no. 4, 6 July 2011 (2011-07-06), pages 1173-1181, XP019935549, ISSN: 1618-2650, DOI: 10.1007/S00216-011-5191-0
- DINCER CAN ET AL: "Multiplexed Point-of-Care Testing - xPOCT", TRENDS IN BIOTECHNOLOGY, ELSEVIER PUBLICATIONS, CAMBRIDGE, GB, vol. 35, no. 8, 26 April 2017 (2017-04-26) , pages 728-742, XP085135996, ISSN: 0167-7799, DOI: 10.1016/J.TIBTECH.2017.03.013
- BRUCH R ET AL: "Clinical on-site monitoring of beta-lactam antibiotics for a personalized antibiotherapy", SCIENTIFIC REPORTS, vol. 7, 9 June 2017 (2017-06-09), XP002784554,
- Curtis D Chin ET AL: "Microfluidics-based diagnostics of infectious diseases in the developing world", Nature Medicine, vol. 17, no. 8, 1 January 2011 (2011-01-01), pages 1015-1019, XP055058272, ISSN: 1078-8956, DOI: 10.1038/nm.2408
- CASTLE JOHN C ET AL: "Mutated tumor alleles are expressed according to their DNA frequency", SCIENTIFIC REPORTS, NATURE PUBLISHING GROUP, US, vol. 4, 1 April 2014 (2014-04-01), XP002774681, ISSN: 2045-2322, DOI: 10.1038/SREP04743
- Geok Soon Lim ET AL: "A lab-on-a-chip system integrating tissue sample preparation and multiplex RT-qPCR for gene expression analysis in point-of-care hepatotoxicity assessment", Lab on a Chip, vol. 15, no. 20, 1 January 2015 (2015-01-01), pages 4032-4043, XP055312781, UK ISSN: 1473-0197, DOI: 10.1039/C5LC00798D
- BRUCH RICHARD ET AL: "Dry Film Photoresist-based Electrochemical Microfluidic Biosensor Platform: Device Fabrication, On-chip Assay Preparation, and System Operation", JOURNAL OF VISUALIZED EXPERIMENTS, JOVE, US, no. 127, 31 August 2017 (2017-08-31), page ArticleNo.:e56105, XP009507875, ISSN: 1940-087X, DOI: 10.3791/56105

## Description

The invention relates to a method and a system for the measurement of multiple analytes from a single sample using a single microfluidic channel along with a measurement cell, wherein the microfluidic channel comprises at least two immobilization areas and at least one spacer area that are serially arranged, and wherein at the site of the immobilization areas analyte-dependent catalyst complexes are formed such that the introduction of an enzyme substrate solution results in the production of target molecules; the amount thereof being detectable as separate peaks, using a measurement cell in combination with a stop-flow technique.

### BACKGROUND OF THE INVENTION

Multiplexing, the simultaneous detection of multiple analytes from a single specimen, has become more and more important in diagnostics, especially with regard to point-of-care testing (POCT) (C. Dincer et al. 2017, S. Spindel et al. 2014, Y.-J. Ko et al. 2008, Q. Fu et al. 2010). Therefore, the implementation of multiplexed bioanalytical devices in a compact, low-cost and sensitive manner is a great and urgent demand. In this context, the miniaturization of microfluidic lab-on-a-chip (LOC) devices by maintaining the sensor sensitivity is fundamental and thus, a matter of research interest (C. D. Chin et al. 2007, A. Bange, et al. 2005, E. K. Sackmann et al. 2014). Herein, the microfluidic chip design plays a decisive role on the analytical performances of biosensor systems. In particular, it reduces the reagent/sample consumption and shortens the assay immobilization as well as the measurement time (C. Dincer et al. 2016, G. M. Whitesides et al. 2006, P. Yager et al. 2006).

In microfluidic biosensor platforms, dealing with enzyme-labeled assays, the size of the immobilization area is a crucial point regarding not only their overall size, but also their system performance as bio- or immunosensors. Either a specific section of the microfluidic capillary or the measurement electrode itself serves as the immobilization site. This brings along non-technical issues (e.g. cost) as well as technical limitations (e.g. sensitivity) on the design and fabrication of such systems, especially in the case of multianalyte platforms.

Chin et al. (Chin et al. 2011) proposed a microfluidic chip (mChip) for an immunoassay on protein markers for the diagnosis of infectious diseases in the developing world. The mChip allows for performing the steps of sample processing, delivery of multiple reagents, signal amplification and final optical detection of benchtop ELISA (enzyme-linked immunosorbent assay) on a miniaturized assay system. By manufacturing the microfluidic cassettes from plastic, productions costs could be reduced. Signal amplification poses a technical problem in optical detection using ELISA assays. Chin et al. proposed, therefore, a reduction of silver ions onto gold nanoparticles in a sandwich ELISA format to overcome an optical background signal. The further disadvantages are its limited flexibility in the assay design, and its moderate multiplexing capability for high-throughput applications.

Another approach for signal amplification for enzyme-mediated microfluidic biosensors is the so called stop-flow technique as described e.g. in US 7691623 B2, which is variant of the previously known flow injection analysis (FIA) (D. Betteridge et al. 1978, J. Ruzicka et al. 1988).

In the stop-flow technique, the fluid flow through a microfluidic channel is stopped for a defined time interval, termed stop-time or stop interval. During this time, an accumulation of products of an enzymatic reaction in an immobilization area occurs. For instance, in case the enzyme glucose oxidase (GOx) has been immobilized, the target molecule hydrogen peroxide (H₂O₂) is produced, when provided with a substrate solution comprising glucose.

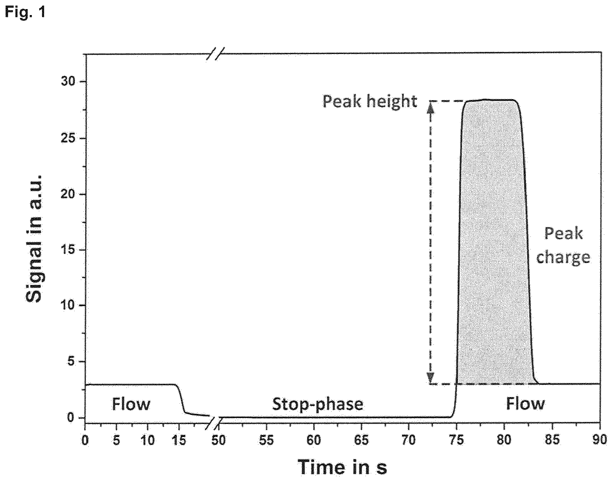

By restarting the fluid flow, the increased concentration of target molecules is subsequently flushed through a detection cell (e.g. an electrochemical cell for the detection of H₂O₂ or an optical cell for different assays). Fig. 1 illustrates the signal profile resulted by the stop-flow technique. Herein, the measured rectangular-like peak signal is directly proportional to the stop-time. The longer the stop duration is, the higher will be the obtained signal amplification. As demonstrated previously by Horak et al., the stop-flow technique, thus, allows for a fast and sensitive detection of analytes independent of their assay format (J. Horak et al. 2014a, J. Horak et al. 2014b, Kling et al. 2016, Bruch et al. 2017).

For the signal evaluation, the stop-flow method offers two different parameters, the peak height and the peak integral (i.e. the charge or the sum of the intensity by electrochemical or optical detection, respectively). In case of a data evaluation based on the peak height, the target molecule concentration profile over the channel cross-section is decisive and thus, leads to a rectangular-like peak signal behavior. This concentration profile depends only on the diffusion coefficient of the target molecule and on the utilized stop-time. Hence, the signal peak height of the stop-flow technique stays constant down to a minimum channel length of the immobilization area. This special feature of the stop-flow technique facilitates a miniaturization of the biosensor geometries, especially regarding channel length, independent of the employed detection method (e.g. electrochemical or optical). Compared to traditional microfluidic biosensor platforms, such an approach of signal amplification enhances the sensor sensitivity and reduces analysis time.

However, a drawback of sensor systems based on the stop-flow techniques as previously described e.g. in Horak et al. 2014a, Horak et al. 2014b, Kling et al. 2016 and Bruch et al. 2017, relates to the cost-sensitive application of measurement cells and fluid control or regulations systems. In particular, when multiple analytes are analyzed, for each analyte a separate system for fluid control and measurement cell is installed in order to allow for an accurate separation of the analyte-specific signals. So far, these limitations have restricted the use of stop-flow approaches for multianalyte detection especially with regard to POCT.

Kling et al. 2016 introduces an electrochemical biosensor with multiplexed microfluidics based on dry film photoresist technology. The device can simultaneously detect up to eight analytes and consists of up to eight discrete immobilization areas. To this end the discrete immobilization areas are not arranged within a single microfluidic channel, but in parallel, separate microfluidic channels. At the end of each channel a separate working electrode (WE) is present that allows for a detection of target molecules produced during an enzymatic reaction (here hydrogen peroxide) as an amperometric peak signal.

Horak et al. 2014b and Ambrecht et al. 2015 disclose a microfluidic chip with a single microfluidic channel for a stop-flow method, but fail to demonstrate a multiplexed detection of multiple analytes.

### OBJECTIVE OF THE INVENTION

An objective of the invention is to provide a method and a system that overcomes the disadvantages of the state-of-the-art devices in providing alternative and/or improved means for measuring multiple analytes in a single sample on a miniaturized microfluidic system. In particular, the invention aimed at realizing a method and a system, using a highly miniaturized microfluidic biosensor, in particular for POCT applications, that is compact, low-cost, and at the same time allows for sufficient sensitivity and rapid sample-to-result times.

The objective is solved by the features of the independent claims. Preferred embodiments of the present invention are provided by the dependent claims.

In a preferred embodiment, the invention relates to a method for the measurement of multiple analytes from a single sample comprising the steps of
a) providing a system comprising
   i. a common inlet configured to receive a fluid stream
   ii. a single microfluidic channel without bifurcation or branching
   iii. a measurement cell
   iv. a channel outlet
      wherein the microfluidic channel comprises at least two immobilization areas and at least one spacer area that are arranged alternating, wherein only at the site of the immobilization areas biorecognition elements are immobilized that possess a binding affinity for an analyte
b) introducing the sample comprising at least two analytes into the microfluidic channel via the common inlet, whereby first analytes immobilize in a first immobilization area and second analytes immobilize in a second immobilization area
c) introducing a fluid comprising a reporter catalyst into the microfluidic channel via the common inlet, whereby the reporter catalyst immobilizes in the respective immobilization areas depending on the presence of analytes to form an analyte-dependent catalyst complex
d) introducing a fluid, containing a substrate for the reporter catalyst
e) stopping the flow of the fluid stream for a stop interval such that in each immobilization area a catalytic reaction may take place to produce target molecules
f) initiating a flow of the fluid stream such that at the measurement cell the amount of target molecules produced in each immobilization area can be detected as separate peak signals.

It is noted that while in a preferred embodiment the methods steps are carried in the order a) through f), the method according to the invention is not be limited to this particular sequence. For instance, it may be preferred to conduct the methods steps b) through d) simultaneously combined or in a different sequence. The method allows for a compact and efficient detection of multiple analytes from a single sample. In contrast to prior art approaches the immobilization areas, in which the analyte-dependent catalysts complexes are formed, are separated by spacer areas, in which preferably no such complexes are present. To this end the spacer areas may preferably be passivated by blocking biomolecules or other substances. When introducing a substrate solution by means of the common inlet, the amount of target molecules produced at the respective immobilization sites directly depends on the presence of analyte-dependent catalysts complexes. Moreover, a regulation of the stop interval, which limits the time for the catalytic reaction, allows for a control of the localized production of target molecules. The inventors have realized that in the context of microfluidics the localized production of target molecules may be employed to facilitate a multiplexing assay in a miniaturized system.

In particular, in this regard the stop-flow technique may exploit the limited diffusion of target molecules along the axial direction of a microfluidic channel to detect their amount as separate peaks using a single measurement cell. It is, therefore, particularly preferred that the provided system comprises only a single measurement cell.

Fluid dynamic behavior is related to the Reynolds number of the flow. As the Reynolds number is reduced, flow patterns depend more on viscous effects and less on inertial effects. Below a certain Reynolds number, e.g., 1, inertial effects can essentially be ignored. Therefore, microfluidic devices do not require inertial effects to perform their tasks, and, therefore, have no inherent limit on their miniaturization due to Reynolds number effects. The fluid dynamic in the microfluidic channel is governed by a laminar and non-turbulent flow. In this regime molecules are transported through by controlled convection, i.e. by initiating a flow using a pump. Mass transport along the axial direction of the channel, i.e. along the length dimension, due to diffusion is limited. Thus, target molecules produced within the immobilization areas do not readily diffuse into the spacer areas, where no target molecules are produced. However, during the fluid flow (especially after the stop-time), the dispersion (so called Taylor dispersion) of these concentration packages of different target molecules should be taken into consideration for the design of the single-channel multianalyte biosensor.

When initiating the fluid flow, the target molecules are transported downstream such that the target molecules produced from a first immobilization area are well separated from target molecules produced in the second immobilization area. Downstream of the at least two immobilization areas and at least one spacer area a measurement cell is situated, which is configured to detect a signal based upon a quantity of passing target molecules. For instance, the target molecules may be hydrogen peroxide and the measurement cell an electrochemical cell. In this case the presence of hydrogen peroxide yields an increase of an electrical current signal, which correlates with the hydrogen peroxide concentration. The stop-flow assay and consecutive arrangement of immobilization areas that are separated by spacer areas, thus, results in the generating of separate peak signals at the measurement cell. Herein, the first detected peak reflects the amount of target molecules produced in the most downstream immobilization area, whereas the last detected peak reflects the amount of target molecules produced in the most upstream immobilization area.

The shape of the peaks allows for a versatile quantification of the produced target molecules. To this end, the peak height or the peak integral may be used. A particular advantage of the stop-flow technique relates to the feature that the signal peak height of the stop-flow technique stays constant down to a minimum channel length of the immobilization area (C. Dincer et al., 2016). Thereby a high signal amplification - i.e. an amplification of the signal of target molecules compared to the presence of analyte-dependent catalyst complexes in the immobilization area - may be achieved. In particular, by miniaturizing the biosensor format signal amplifications of more than about 1 00-fold can be reached compared to constant flow measurements.

By means of the design, a compact microfluidic system for multiplexing a single sample can be constructed. Herein, the number of analytes detectable is equal to the number (N) of immobilization areas separated by N-1 spacer areas. The total length of the channel is thus N times the length of an immobilization area plus N-1 times the length of a spacer area.

Moreover, due to the consecutive design of the immobilization areas along a microfluidic channel a single measurement cell is sufficient for an analysis of arbitrary many analytes. Thus, in case of an exemplary design for multiplexing 4 analytes the method according to the invention only needs one measurement cell, while previous approaches with a parallel arrangement of immobilization areas in a microfluidic channel network have necessitated 4 measurement cells. Additionally, the serial microfluidic chip design guarantees the same flow conditions in all immobilization areas and the same sensor performance for different analytes (because of the single measurement cell) in comparison to the parallel approach.

Furthermore, the design of the microfluidic channel can be chosen according to the preferred dimensions of the system. For instance, it may be preferred to use a rectilinear microfluidic channel, in which the immobilization areas are consecutively arranged along a straight line. However, also curved designs of the channel can be realized, resulting in a reduced channel length of the overall system. According to the invention the microfluidic channel is a single continuous channel without bifurcation or branching.

Different techniques for fabricating a microfluidic channel with the desired dimension may be used. Exemplary techniques include lithography, such as photolithography or depth lithography, thin film deposition, etching techniques, such as wet etching or dry etching, casting techniques, e.g. soft lithography, injection molding, bonding techniques including silicon direct bonding, anodic bonding or glass bonding, plasma-activated bonding or maskless patterning techniques such as laser micromachining, inkjet printing or 3D printing. The details of these and other suitable techniques are known to the person skilled in the art and reviewed e.g. in M. Leester-Schädel et al. 2016. Examples of suitable materials for the fabrication of the microfluidic channel include, for instance, polydimethylsiloxane (PDMS), glass, silicon, metals, non-metal thin films, SU-8 or polyimide.

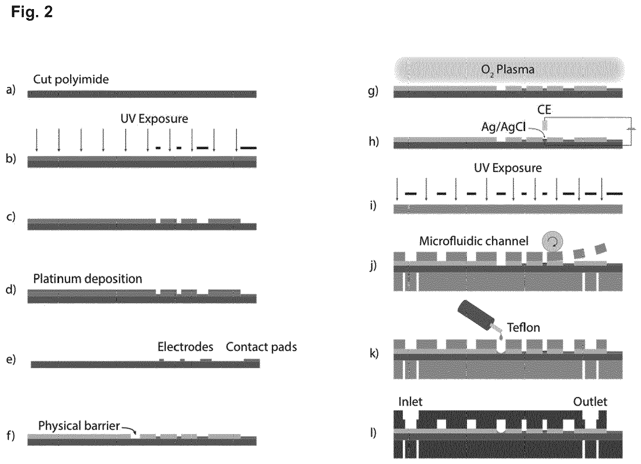

In a particularly preferred embodiment, the system, comprising the microfluidic channel, is manufactured by means of photolithography, such as a dry film photoresist (DFR) technology. In this case, the fabrication of the microfluidic channel together with a measurement cell, e.g. an electrochemical cell, may be realized on wafer-level, using a polyimide substrate and photolithographic techniques, employing multiple DFR layers. An exemplary protocol for such a fabrication method is disclosed in Example 1 and Fig. 2. Such a fabrication method allows for a particular cost-efficient manufacturing of robust devices and an easily implemented method.

Advantageously the method does not pose any intrinsic limitations on the samples that can be multiplexed. Instead the method relies simply on the formation of analyte-dependent catalyst complexes specifically in the immobilization areas at a geometrically distinct location from the at least one spacer area.

To form analyte-dependent catalyst complexes at the site of the immobilization area biorecognition elements are adsorbed or immobilized that allow for a binding, i.e. capturing, of the analyte at these sites.

In a preferred embodiment of the invention the biorecognition elements are selected from a group consisting of nucleic acids, preferably RNA and/or DNA oligonucleotides, antibodies, peptides, proteins, aptamers, molecularly-imprinted polymers or even whole cells. It is particularly preferred that the biorecognition elements are chosen to exhibit a high specific binding to the respective analytes. For instance, in case of an antigen as an analyte the biorecognition element may refer to an antibody specific to the antigen. By immobilizing different antigen-specific antibodies at each of the immobilization sites, multiple analytes can be selectively captured.

In a preferred embodiment of the invention, the method is thus characterized in that in the first immobilization area first biorecognition elements are immobilized with a specific binding affinity for first analytes, whereas in the second immobilization area second biorecognition elements are immobilized with a specific binding affinity for second analytes. A person skilled in the art knows how to choose biorecognition elements for the desired analytes. In addition to the above-mentioned example of an antibody-antigen pair, e.g. also DNA oligonucleotides may be immobilized. DNA oligonucleotides allow for the specific capturing of nucleic acids in a sample due to the high specificity of the sequence dependent hybridization process.

By preparing the immobilization areas accordingly multiple analytes immobilize to distinct immobilization sites after an introduction of the sample through the common inlet. To prepare for the readout using a stop-flow assay, analyte-dependent catalyst complexes are to be formed at the immobilizations sites. To this end the person skilled in the art is aware of a number of different assays.

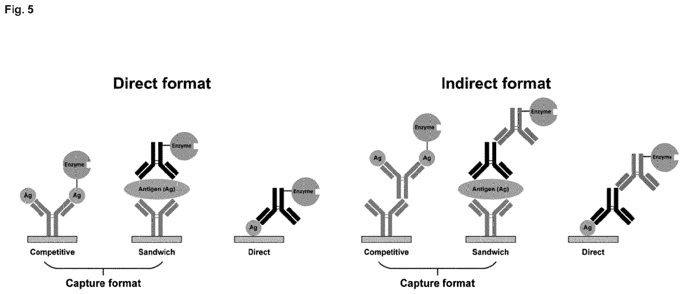

In a preferred embodiment of the invention the analyte-dependent catalyst complexes are formed by introducing reporter catalysts conjugated with analyte-specific binding partners and/or introducing in an intermediate step analyte-specific binding partners and subsequently introducing reporter catalysts conjugated with a binding partner for said analyte-specific binding partners. The preferred embodiment is also known as a sandwich assay in which the biorecognition elements form one layer of the sandwich, while a complex comprising the catalyst forms a second layer of the molecular sandwich (see Fig. 5). Herein the analyte, e.g. an antigen, should preferably comprise at least two binding sites enabling a binding to the biorecognition element as well as to a binding partner of the catalyst complex. To ensure a binding of the catalyst to the analyte, the catalyst may be conjugated with a binding partner for said analyte, i.e. an antigen-specific antibody in case of an antigen as an analyte. This assay is also referred as a direct sandwich assay. Alternatively, in an indirect assay an intermediate binding partner may be introduced, which could be for instance a secondary antibody that recognizes the analyte. In an indirect assay the catalyst is preferably conjugated to a binding partner, e.g. an antibody, that specifically recognizes the intermediate binding partner, e.g. the secondary antibody. In both assay formats - indirect or direct sandwich - an analyte-dependent catalyst complex is formed. Thereby, the reporter catalyst specifically immobilizes to the immobilization areas depending on the number of immobilized analytes being present at these sites.

Since for the subsequent stop-flow readout it is advantageous to introduce a solution with a single substrate type, it is preferred that identical catalysts immobilize to the different immobilization areas. The specificity for the multiplexing assay may be introduced by conjugating a catalyst to multiple analyte-specific binding partners in a direct assay. Alternatively, multiple analyte-specific intermediate binding partners may be introduced into the microfluidic channel.

In a further preferred embodiment of the invention the method is characterized in that for each native analyte that is to be measured in the sample a labeled analyte is added, wherein the label constitutes a binding partner to allow for the formation of an analyte-dependent catalyst complex. Said preferred embodiment constitutes a competitive assay for the measurement of analytes within a sample (see Fig. 5). Herein, the analyte, e.g. an antigen, is preferably conjugated to one member of a binding pair, for instance biotin. The hence labeled analyte is added to the sample in a known concentration. For the multianalyte assay many different labeled analytes may be added to the sample. It is particularly preferred that all of the added analytes are labelled with the same member of a binding pair (e.g. biotin) in order to facilitate the subsequent immobilization of the catalyst. After introducing the sample, the native and labeled analytes will compete for the binding sites of analyte-specific biorecognition elements at each of the immobilization areas.

For forming the analyte-dependent catalyst complex in a direct assay, reporter catalysts are introduced that are conjugated to the second member of the binding pair. For instance, in case biotinylated analytes were added to the sample catalysts linked to streptavidin may be used. Alternatively, in an indirect assay an intermediate binding partner, e.g. a secondary antibody conjugated to streptavidin, may be introduced into the microfluidic channel. Subsequently, catalysts conjugated with a binding partner specific for the intermediate binding partner, e.g. the secondary antibody, are added. In the competitive assay, the quantity of immobilized analyte-specific catalyst complexes is inversely proportional to the amount of unlabeled, i.e. native, analytes in the sample.

For a readout of the quantity of the analyte-dependent catalyst complexes at the immobilization sites a substrate solution is introduced, using a stop-flow technique. Herein, during the stop interval detectable target molecules are produced, wherein the amount of target molecules directly depends on the reporter catalysts being present. To this end, a variety of different reporter catalysts can be chosen.

In a preferred embodiment of the invention, the reporter catalyst is a reporter enzyme preferably selected from a group consisting of glucose oxidase, horseradish peroxidase, alkaline phosphatase, Chloramphenicol acetyltransferase, β-galactosidase and β-glucuronidase. These preferred enzymes are characterized by an efficient catalyzation of a reaction yielding readily detectable target molecules upon provision of a solution comprising a substrate. For instance, glucose oxidase as a reporter enzyme produces gluconate and hydrogen peroxide upon provision of a substrate solution comprising glucose. Herein, the target molecule hydrogen peroxide can be amperometrically detected at a working electrode in an electrochemical cell.

Moreover, horseradish peroxidase (HRP) catalyzes the conversion of chromogenic substrates as e.g. 3,3',5,5'-Tetramethylbenzidine (TMB), 3,3'-Diaminobenzidine (DAB), 2,2'-azino-bis(3-ethylbenzothiazoline-6-sulphonic acid (ABTS) in the presence of hydrogen peroxide into coloured products. These coloured products may serve as target molecules detectable by an optical cell. Similarly, β-galactosidase catalyzes the cleavage of the substrate 5-bromo-4-chloro-3-indolyl-β-D-galactopyranoside (X-Gal), which consists of galactose linked to a substituted indole. The catalytic reaction yields galactose and 5-bromo-4-chloro-3-hydroxyindole that spontaneously dimerizes and is oxidized into 5,5'-dibromo-4,4'-dichloro-indigo. The latter may serve as a target molecule that can be readily detected in an optical cell due to its intense blue colour.

A person skilled in the art understands that a variety of reporter enzymes may be preferably used in the assays as described herein and will be able to accordingly choose the substrate solution. For references on enzyme reporters as well as assays to form analyte-dependent catalyst complex, the person skilled in the art may rely on reports on recent developments as well as standard literature in the field as e.g. Immunoassay Handbook: Theory and Applications of Ligand Binding, ELISA and Related Techniques 4th Edition, Ed. David Wild, Elsevier Oxford UK, 2013.

In a preferred embodiment of the invention, the measurement cell is an electrochemical cell comprising one or more working electrode(s) and a common counter electrode. It is particularly preferred that the electrochemical cell comprises one or more working electrode(s) also known as the sensing or redox electrode, a common reference electrode and a common counter or auxiliary electrode. The reference electrode may be made for instance from silver/silver chloride (Ag/AgCl) and is kept at a short distance from the reaction site in order to maintain a known and stable potential. The working electrode serves as the transduction element in the biochemical reaction, while the counter electrode establishes a connection to the electrolytic solution so that a current can be applied to the working electrode. It is preferred that the electrodes are both conductive and chemically stable. To this end, novel metals, such as platinum, gold, or carbon-based materials (e.g. graphite, graphene) and metal oxides (e.g. iron oxide) and semiconductor (e.g. zinc oxide) compounds may be preferred as electrode material or modification depending on the target molecules. As a readout of the electrochemical reaction the cell typically generates a measurable current (amperometric), a measurable potential or charge accumulation (potentiometric) or measurably alters the conductive properties of a medium (conductometric) between the electrodes.

In amperometric devices, the measureable current results from the oxidation or reduction of an electroactive species, i.e. the target molecules. A person skilled in the art knows how to design electrochemical cells suited for the detection of desired target molecules. For reference of further possible electrochemical detection schemes the person skilled in the art may rely on a variety of literature reports and reviews as e.g. Mehrvar et al., 2004 or Grieshaber et al., 2008.

In a further preferred embodiment of the invention, the measurement cell is an optical cell comprising a light source and a photo detector. In this embodiment, optical properties of the target molecules in altering the electromagnetic field of the light source are exploited. Suitable optical detection schemes include for instance fluorescence, absorbance or luminescence measurements as well as surface plasmon resonance-based optical detection methods. The optical detection methods are characterized by a high specificity and correlation with the target molecule concentration. Suitable light sources include e.g. light- emitting diodes (LEDs), lamps, laser or laser diodes. In addition, optical fibers, gradient refractive index lenses or diffractive elements may be used to construct a compact optical cell. As a photodetector, e.g. photo diodes (e.g. avalanche photo diodes (APD)), photo multipliers (PMT) or charge coupled devices (CCD) may be employed. Advantageously by combining the stop-flow technique with an optical cell a compact low-cost multiplexing analyte system may be provided. In this context, a variety of optical detections schemes may be advantageously implemented including the ones disclosed in Simon et al, 2015 and Kuswandia et al. 2007.

Suitable electrochemical as well as optical detection schemes result in the detection of signal peaks at the measurement cell that precisely reflect the concentration of target molecules produced in the respective immobilization area. Furthermore, calorimetric and magnetic signal detection techniques could be also employed. Thereby, a reliable deduction on the distribution of analytes may be achieved.

In a further preferred embodiment, the amount of target molecules is determined based upon the height and/or integral of the signal peak. Both parameters are directly proportional to the stop interval and therefore, can reflect the amount of target molecules produced by the analyte-dependent catalyst complex. The peak height depends on the maximum target molecule concentration, its diffusion coefficient as well as the applied stop-time. The longer the stopping time is, the higher is the measured signal response. For this reason, the gauged peak height remains constant down to a minimum length of the immobilization capillary. This special feature of the stop-flow technique allows a drastic decrease in the dimensions of the microfluidic channel, particularly the length of the immobilization area, while ensuring a high sensitivity of the sensor.

In a further preferred embodiment of the invention, the microfluidic channel comprises at least 4, most preferably more than 6 immobilization areas and a number of spacer areas, which are equal to the number of immobilization areas minus 1. Advantageously the alternating arrangement of the immobilization areas and spacer areas allows for a compact design, while enabling the detection of more than 4, more than 6 and even more than 8 different analytes in a single sample. In this preferred embodiment, the inventive feature of the method disclosed herein is exploited in that multiple peaks representing the produced target molecules separate well in an arrangement of a microfluidic channel as described herein.

In particular, in combination with electrochemical and/or optical cells a reliable, fast and precise measurement of multiple analytes in a single sample can be achieved.

In a further preferred embodiment of the invention the cross-sectional dimension of the microfluidic channel is in between 10 nm and 10 mm, preferably between 0.2 µm and 2000 µm, most preferably between 10 µm and 1000 µm. The term cross-sectional dimension refers preferably to the inner characteristic dimension of the microfluidic channel that is perpendicular to said fluid flow. For instance, the microfluidic channel may have an inner surface with a circular shaped cross-section. In this case the inner cross-sectional dimension of the channel is equal to the inner diameter of the channel. However, the channel may also have an inner surface with a square shaped cross-section for which the cross-sectional dimension would preferably refer to the length of said square. The preferred cross-sectional dimensions result in peak shapes of the detected target molecule distribution that correlate particularly well with the amount of analyte-dependent catalyst complexes. The preferred cross-sectional dimensions yield, therefore, particular accurate analysis results.

In a further preferred embodiment of the invention, the length of each of the immobilization areas is in between 1 mm and 100 mm, preferably between 5 mm and 50 mm, most preferably between 15 mm and 25 mm. These dimensions present an optimal compromise in yielding an excellent signal amplification, while maintaining a short overall channel length.

In a further preferred embodiment of the invention the length of each of the spacer areas is in between 1 mm and 100 mm, preferably between 5 mm and 50 mm, most preferably between 15 mm and 25 mm. It is particularly preferred that the length of the spacer areas is on the order of the length of the immobilization areas, as this allows the most efficient usage of the channel dimension, while ensuring optimal peak signal separation and analyte detection.

In a further preferred embodiment of the invention, the length of the stop interval is more than 5 sec, preferably in between 5 sec and 30 min, more preferably between 10 sec and 5 min, most preferably between 30 sec and 2 min. Experiments showed that for the aforementioned preferred parameter regimes an optimal balance of signal amplification and peak separation may be reached.

These stop intervals are particularly suited for the aforementioned preferred embodiments of the dimension of the immobilization area, spacer area and cross-sectional dimension in the microfluidic channel. That means it is particularly preferred to allow for a stop interval of in between 5 sec and 30 min, more preferably between 10 sec and 5 min, most preferably between 30 sec and 2 min for microfluidic channels with a cross-sectional dimension of 10 nm and 100 mm, preferably between 0.2 µm and 2000 µm, most preferably between 10 µm and 1000 µm, wherein the length each of the at least immobilization areas is in between 1 mm and 100 mm, preferably in between 5 mm and 50 mm, most preferably in between 15 mm and 25 mm and the length of the at least one spacer area is in between 1 mm and 100 mm, preferably between 5 mm and 50 mm, most preferably between 15 mm and 25 mm.

In a further preferred embodiment of the invention, the microfluidic channel comprises individual inlets for each immobilization area such that for each immobilization area a fluid comprising the biorecognition elements may be introduced by said individual inlets. The preferred embodiment thus allows for a particularly fast, easy and reliable preparation of immobilization areas in which analyte-specific biorecognition elements are immobilized or adsorbed.

In a particularly preferred embodiment of the invention, the at least two immobilization areas and the at least one spacer area are separated by stopping barriers. Thereby, an additional separation between two immobilization areas and a spacer area can be achieved which effectively impedes the cross-talk between these distinct sections of the microfluidic channel. Moreover, when biorecognition elements for the preparation of the immobilization areas are introduced by individual inlets the stopping barriers allow constraining the distribution of the biorecognition element to the immobilization areas.

By combining individual inlets and stopping barriers for each immobilization are, the binding of biorecognition elements only in a particular immobilization section can be ensured, which augments the robustness of the detection method. To this end, the fluid comprising the biorecognition elements may passively spread on or wet the immobilization area, while stopping barriers impede a fluid spreading outside the designated sections.

In a particularly preferred embodiment of the invention, the stopping barriers are hydrophobic sections of the microfluidic channel and/or geometric structures configured to impede capillary spreading of a fluid. Hydrophobic materials have preferably little or no tendency to adsorb water and water tends to bead on their surfaces in discrete droplets. Hydrophobic materials possess low surface tension values and lack active groups in their surface chemistry for formation of hydrogen-bonds with water. Examples of particularly suited hydrophobic materials or coatings include hydrophobic fluoropolymers, in particular polytetrafluoroethylene (PTFE), fluorinated ethylene propylene (FEP) or perfluoroalkoxy alkanes (PFA) as well as superhydrophobic coatings such as manganese oxide polystyrene (MnO₂/PS) nanocomposite, zinc oxide polystyrene (ZnO/PS) nanocomposite, precipitated calcium carbonate, carbon nanotube structures and/or silica nanocoatings.

Including a hydrophobic stopping barrier into the microfluidic channel constitutes a deviation from the teaching of the prior art, which aims to provide microfluidic channels with particularly good spreading properties. However, in the context of the preparation of immobilization areas, the stopping areas proved efficient. Moreover, the stopping barriers did not result in a distortion of the consecutive peak signals stemming from the target molecules. Instead the stopping barriers additionally support the peak separation and thus, distinction of the different analytes.

Different geometric structures or physical barriers may serve to impede the spreading of a fluid. For instance, freeze-dried sugar may be used to form an effective physical barrier.

In a further embodiment of the invention, the system comprises an additional measurement cell at the at least one spacer area. While the arrangement of the immobilization and spacer areas allows to only use a single measurement cell for detecting multiple analytes in a single sample. It may also be preferred to install additional measurement cells at each of the spacer areas. Thereby, the concentration of the target molecules produced in an immobilization area can be detected directly in the adjacent downstream spacer area. The measurement cells at the spacer areas are preferably optical cells or electrochemical cells. In case of an electrochemical cell it may be particularly preferred to use multiple working electrodes at each of the at least one spacer areas together with one or more reference and counter electrodes. By applying a voltage to all electrodes, the current at each of the sequential electrodes can be measured and the concentration of produced target molecules deduced.

In a further aspect the invention relates to a system for multiplexing a sample comprising
i. a common inlet configured to receive a fluid stream
ii. a single microfluidic channel without bifurcation or branching
iii. a measurement cell
iv. a channel outlet
v. control means for introducing the fluid into the common inlet and regulating the fluid stream within the microfluidic channel, wherein the control means include flow inducing or controlling means and a processor unit, wherein the processor unit allows for a combined regulation of the flow inducing or controlling means and the measurement cell
characterized in that
the microfluidic channel comprises at least two immobilization areas and at least one spacer area that are arranged alternating, wherein at the site of the immobilization areas biorecognition elements are immobilized that possess a binding affinity for an analyte and wherein the control means are configured to allow for a stop-flow measurement for gauging the production of target molecules by analyte-dependent catalyst complexes at the site of the immobilization areas.

Technical features that have been disclosed for the method according to the invention preferably also apply for the system according to the invention. A person skilled in the art will, therefore, recognize that preferred features of the method according to the invention can be advantageously used as well in the context of the system according to the invention. In particular, the system according to the invention is particularly suited for performing the method according to the invention as well as preferred embodiments thereof.

To this end, the system comprises preferably control means for a stop-flow protocol/technique/readout for measuring the amount of target molecules produced by analyte-dependent catalyst complexes. Such control means preferably include flow inducing and controlling means including peristaltic or reciprocating pumps, syringes, electricity or piezo elements. With these flow-inducing means, the initiation and stopping of a fluid stream within the microfluidic channel can be achieved with a high precision. Furthermore, it is particularly preferred that the control means comprise an automatic controller and processor unit which allows for combined regulation of the flow inducing means as well as the measurement cell. For instance, it is particularly preferred that the control means are configured to introduce a substrate solution, to allow for a production time of the target molecules during a predefined stop interval, subsequently to initiate the flow and at the same time trigger a signal acquisition at the measurement cell. To this end, a hardware or software implemented method may be installed on the controller and/or processor unit, wherein the computer implemented method is configured to control the fluid inducing means and components of the measurement cell. It is particularly preferred that the controller and processor unit is a computer, a notebook or similar device. It may however also be preferred that the controller and processor unit is a microchip and thereby allows for a miniaturization of the system. The system comprises preferably in addition a data storage unit that allows for a storage of the acquired data and subsequent analysis of the signal peaks e.g. by analyzing the peak height and/or integral.

The system according to the invention is further characterized in that the control means are configured to
a) introduce the sample comprising at least two analytes into the microfluidic channel via the common inlet, whereby first analytes immobilize in a first immobilization area and second analytes immobilize in a second immobilization area
b) introduce a fluid comprising a reporter catalyst into the microfluidic channel via the common inlet, whereby the reporter catalyst immobilizes in the respective immobilization areas depending on the presence of analytes to form an analyte-dependent catalyst complex
c) introduce a fluid containing a substrate for the reporter catalyst
d) stop the flow of the fluid stream for a stop interval such that in each immobilization area a catalytic reaction may take place to produce target molecules
e) initiate a flow of the fluid stream such that at the measurement cell the amount of target molecules produced in each immobilization area can be detected as separate peak signals.

It is particularly preferred the configuration of the control means refers to an automatic procedure implemented on the processing and/or control unit that is able to regulate the flow inducing means and the measurement cell. However, certain steps may also be performed manually while others are performed automatically. For instance, it may be preferred that the control means allow for a manual introduction of the sample, while the subsequent readout of the formed analyte-dependent catalyst complexes in a stop-flow assay is performed automatically.

The person skilled in the art further understands that technical features as well as their advantages disclosed for the method described herein equally apply to the system. For instance, for the method preferred dimensions of the spacer length, immobilization area and cross-sectional dimension of the microfluidic channel have been disclosed as they lead to a particular fast and confident detection of multiple analytes in a sample. For a person skilled in the art, it is therefore evident that it may be particularly preferred to design the microfluidic channel as well as the geometric dimension and arrangement of the immobilization areas and spacer areas according to the same preferred design rules. Furthermore, as for the method while in preferred embodiments the control means are configured to carry out the steps in the order a) through d), the system is not be limited to this particular sequence. For instance, it may be preferred to conduct the steps a) through d) simultaneously combined or in a different sequence.

Furthermore, it has been disclosed that for the method described herein it may be preferred that the biorecognition elements are immobilized by the combined effect of individual inlets for each immobilization area and stopping barriers limiting fluid spreading. A person skilled in the art understands that hence a system comprising such individual inlets together with stopping barriers separating the alternating at least two immobilization areas and spacer area is equally preferred. Furthermore, in a particular preferred embodiment the system comprises only a single measurement cell that as described herein allows for the readout of multiple analytes in a single sample.

### DETAILED DESCRIPTION OF THE INVENTION

The present invention is directed to the measurement of multiple analytes in a sample, using a stop-flow technology. Before the present invention is described with regards to the examples, it is to be understood that unless otherwise indicated this invention is not limited to specific analytes as such may vary. It is also to be understood that the terminology used herein is for the purpose of describing particular embodiments only and is not intended to limit the scope of the present invention.

The term "analyte" refers to a substance to be detected, quantified or otherwise assayed by the method of the present invention. Typical analytes may include, but are not limited to proteins, peptides, nucleic acid segments, carbohydrates, lipids, antibodies (monoclonal or polyclonal), antigens, oligonucleotides, specific receptor proteins, ligands, molecules, cells, microorganisms and fragments and products thereof, or any substance for which attachment sites, binding members or receptors (such as antibodies) can be developed.

The term "analyzing" herein refers to detecting the presence, qualitatively identifying, or quantitatively measuring the amount, concentration, or activity of analytes.

The term "sample" as used herein refers to a solution comprising at least one analyte, preferably two or more analytes. Examples of samples include biological fluids such as serum, plasma, urine, tear, cells, cell mixtures, cell culture supernatants, or cell lysates containing one or more biological target molecules. Furthermore, samples can also comprise any conditioning reagents (e.g., permeablizing reagents) needed to render analytes soluble and accessible to detection and quantification. Such conditioning reagents may be added to the sample at any time before or after the sample is added to the devices of the present invention.

The term "assay" as used herein refers to a procedure used for the quantitative or qualitative analysis of an analyte. Examples of assays include immunological assays, antigen-antibody assays, enzyme-linked immunosorbent assays, oligonucleotide hybridization assays and the like.

The term "immobilized biorecognition element" refers to any substance capable of binding specifically an analyte, such as an antibody, receptor, lectin, nucleic acid or binding protein, which has been immobilized by attachment to an appropriate support. Also, in some instances, the immobilized biorecognition element may simply be comprised of a solid support matrix to which an analyte may bind without the aid of an intermediary substance. The biorecognition elements may also be referred to as capture reagents, since they preferably bind (i.e. capture) the analytes to be measured.

Preferably the biorecognition elements are biomolecules. As used herein the term "biomolecules" preferably refers to molecule that are present in living organisms or may be used in the metabolism of living organisms, including large macromolecules such as proteins, carbohydrates, lipids, and nucleic acids, as well as small molecules such as primary metabolites, secondary metabolites, and natural products. Within the scope of the invention, biomolecules may refer to natural products, semisynthetic molecules or synthetic molecules. Herein, semisynthetic biomolecules refer to molecules that are molecular versions of the biomolecules producible by living organisms and subsequently modified to achieve desired properties, whereas synthetic biomolecules molecules are molecules that resemble the functionally properties of natural biomolecules, in particular in regard to binding sites, but are produced using methods of chemically synthesis.

Particularly preferred biorecognition elements refer to nucleic acids, preferably RNA and/or DNA oligonucleotides, antibodies, peptides, proteins, aptamers, molecularly-imprinted polymers or even whole cells that have a binding affinity for the analytes. In analyzing multiple analytes, it is particularly preferred that a first type of immobilized biorecognition elements specifically binds to a first type of analyte, while a second type of immobilized biorecognition elements specifically binds to a second type of analyte.

By "protein", a sequence of amino acids is meant for which the chain length is sufficient to produce the higher levels of tertiary and/or quaternary structure. "Peptides" preferably refer to smaller molecular weight proteins.

The term "antibody" as used herein covers monoclonal antibodies (including full-length monoclonal antibodies), polyclonal antibodies, multispecific antibodies (e.g., bispecific antibodies), and antibody fragments so long as they exhibit the desired biological activity.

"Antibody fragments" comprise a portion of a full-length antibody, generally the antigen binding or variable region thereof. Examples of antibody fragments include Fab, Fab', F(ab')₂, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al, Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. NatL. Acad Sci. USA 81 :6851-6855 (1984)).

The term "binding partner" preferably refers to one member of a binding pair, wherein the term "binding pair" includes any of the class of immune-type binding pairs, such as antigen/antibody or hapten/anti-hapten systems; and also any of the class of nonimmune-type binding pairs, such as biotin/avidin; biotin/streptavidin; folic acid/folate binding protein; complementary nucleic acid segments; protein A or G/immunoglobulins; and binding pairs which form covalent bonds, such as sulfhydryl reactive groups including maleimides and haloacetyl derivatives, and amine reactive groups such as isotriocyanates, succinimidyl esters and sulfonyl halides.

The term "analyte-dependent catalyst complex" refers to an immobilized complex, which is formed in response to the presence of an analyte. The complex will contain an analyte and a catalyst.

As used herein the term "catalyst" generically describes a molecule which increases the rate of a biological or chemical reaction, but which is not necessarily consumed by the reaction. The presence of catalysts increases the production of target molecules, when presented with a substrate solution. As part of an analyte-dependent catalyst complex the catalytic function allows for a deduction of the amount of analytes based upon the quantity of produced target molecules. The catalysts are therefore also referred to as "reporter catalysts". It is particularly preferred that the reporter catalysts are reporter enzymes.

Enzymes preferably useful in the present invention may comprise any protein that exhibits enzymatic activity in catalyzing the reaction of a substrate to yield target molecules and can be thus used as reporter enzymes. In particular reporter enzymes as preferred herein are suited for a signal amplification of the presence of analytes at the sites of the immobilization areas. Examples of such reporter enzymes include glucose oxidase, luciferase, alkaline phosphatase, β-galactosidase, β-glucuronidase, carboxylesterase, lipases, phospholipases, sulphatases, peptidases, proteases peroxidase, glucose-6-phosphate dehydrogenase, carboxyl esterase or creatine kinase or the like. A person skilled in the art understands that other reporter enzymes may be used and will be able to choose the substrate solution accordingly.

As used herein the term "substrate" refers to one or molecules upon which the catalysts act to produce target molecules. For instance, for the reporter enzyme glucose oxidase a suitable substrate solution comprises glucose, wherein the glucose oxidase catalyzes the oxidation of the glucose to yield hydrogen peroxide as a target molecule.

As used herein the term "target molecule" refers to molecules that are produced in the catalytic reaction of the catalyst acting upon the substrate. Since the quantity of produced target molecules proportionally correlates with the quantity of catalysts, a measurement of target molecules allows for a deduction of the quantity of immobilized catalysts within the immobilization area.

The "immobilization area" refers to a section of the microfluidic channel in which analyte-dependent catalyst complex are formed. The immobilization area thus preferably refers to an area or site of the microfluidic channel, wherein analytes and the reporter catalysts immobilize. To this end, it is preferred that at the site of the immobilization area biorecognition elements are immobilizes that bind (i.e. capture) the analytes to be measured. Depending on the assay the formation of analyte-dependent catalysts at these sites is achieved by a direct or indirect binding of the catalysts to the analytes. A person skilled in the art knows how to choose binding pairs and how to choose and or prepare analytes, catalysts or intermediate binding partners in order to ensure that in the immobilization area catalysts will immobilizes depending on the presence of analytes within the sample.

The immobilization areas are distinct from the "spacer areas" in that at the site of the spacer areas preferably no formation of analyte-dependent catalysts complex occurs. To this end, the spacer areas may be passivated to impede the specific or unspecific binding of analytes, catalysts or intermediate binding partners of an analyte-dependent catalyst complex. Passivation preferably refers to a coating of the spacer areas to render the sites inert and inhibit an undesired adherence or binding of biomolecules. Passivation of the spacer areas may be achieved e.g. using polyethylene glycol (PEG) or Bovine serum albumin (BSA).

A "channel" as used herein, means a feature on or in an article (substrate) that at least partially directs a flow of a fluid. The channel can have any cross-sectional shape (circular, oval, triangular, irregular, square or rectangular, or the like) and can be covered or uncovered. In embodiments where it is completely covered, at least one portion of the channel can have a cross-section that is completely enclosed, or the entire channel may be completely enclosed along its entire length with the exception of its inlet(s) and/or outlet(s). A channel may also have an aspect ratio (length to average cross-sectional dimension) of at least 2:1, more typically at least 3: 1, 5:1, 10:1, 15:1, 20:1, or more. An open channel generally will include characteristics that facilitate control over fluid transport, e.g., structural characteristics (an elongated indentation) and/or physical or chemical characteristics (hydrophobicity vs. hydrophilicity) or other characteristics that can exert a force (e.g., a containing force) on a fluid. The fluid within the channel may partially or completely fill the channel.

Herein the term "microfluidic channel" refers to a channel having a cross-sectional dimension of less than 10 mm preferably less than 5000 µm most preferably less than 1000 µm and a ratio of length to largest cross-sectional dimension of at least 3:1 preferably at least 5:1 and most preferably at least 10:1.

In the context of the invention the "axial" direction of the channel as well as the "length" of a channel refers to the dimension along the fluid flow within said channel, whereas the "lateral" or "traverse" direction refers to the dimension orthogonal to the fluid flow.

Furthermore, in line with the fluid flow "upstream" preferably refers to a location in direction of the common channel inlet, whereas "downstream" preferably refers to a direction towards the common channel outlet.

The term "flow inducing means" encompasses devices which have the ability to induce flow. Flow may be induced by negative pressure, positive pressure, electrophoretically or osmotically. This may be accomplished by peristaltic or reciprocating pumps, syringes, electricity, or piezo elements. In a preferred embodiment, the flow inducing means include the ability to reverse the flow direction. For example, a device capable of exerting both positive and negative pressure can induce flow by positive pressure and can reverse flow by negative pressure.

The term "stop-flow" means stopping or temporarily halting the fluid flow through the microfluidic channel to allow the production of target molecules during a catalytic reaction within the immobilization areas. The term "stop interval" refers preferably to the period of time for which the fluid stream in the microfluidic channel is halted to allow for said catalytic reaction.

It must be noted that as used herein and in the claims, the singular forms "a," "an" and "the" include plural referents unless the context clearly dictates otherwise. Thus, for example, reference to "a catalyst" includes two or more catalysts; reference to "an antibody" includes two or more antibodies, and so forth.

### FIGURES

The following figures are presented in order to describe particular embodiments of the invention, by demonstrating a practical implementation of the invention, without being limiting to the scope of the invention or the concepts described herein.

### Short description of the figures:

- **Fig. 1:**: Illustration of a signal response obtained from a stop-flow measurement
- **Fig. 2:**: Graphical illustration of the different fabrication steps for a microfluidic biosensor platform as example system.
- **Fig. 3**: Illustration of the operating principle of an electrochemical microfluidic platform as example system.
- **Fig. 4:**: Electrochemical signal readout of the enzyme linked assay, using the stop-flow technique.
- **Fig. 5**: Illustration of different assays for the formation of an analyte-dependent catalyst complex at the site of the immobilization areas.
- **Fig. 6**: Chip design for multiplexing with multiple immobilization areas and spacer areas arranged alternating in a single microfluidic channel.
- **Fig. 7**: Results of numerical simulations for a microfluidic multiplexed biosensor for a dual analyte detection.

### Detailed description of the figures:

**Figure 1****:** Illustration of a signal response obtained from a stop-flow measurement. For the data evaluation, either the maximum height or the integral of the rectangular-shaped peak signal can be utilized. Both signal parameters are proportional to the period or the stop-phase.
**Figure 2****:** Graphical illustration of the different fabrication steps of a microfluidic biosensor platform as example system. a) Polyimide substrate double-sided covered with 18 µm of copper. b) Copper free polyimide wafer after the copper etching. c) Spin coating and developing of a positive photoresist for lift-off process, d) Physical vapor deposition of platinum onto the substrate. e) Lift-off process to remove the excess photoresist. f) Spin coating of SU-8, forming an insulation layer. g) O₂ Plasma process to remove SU-8 residues on the Pt electrodes. h) Galvanic deposition of the Ag/AgCI reference electrode. i) UV exposure and development of different DFR layers. j) Lamination of the DFR layers onto the PI wafer, k) Dispensing solved polytetrafluoroethylene, forming a hydrophobic stopping barrier between the immobilization capillary and the electrochemical cell. I) Final electrochemical, microfluidic biosensor.

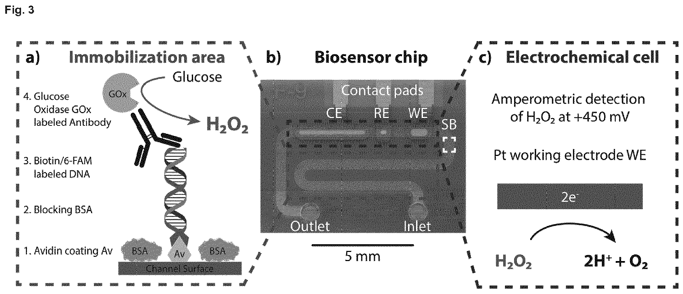

**Figure 3****:** Illustration of an operating principle of an electrochemical microfluidic platform. a) Schematics of a model assay, based on avidin. b) Photograph of the microfluidic biosensor, showing its main elements, including the counter electrode CE, the reference electrode RE and the working electrode WE. The immobilization area and electrochemical cell are highlighted by the dashed rectangles, respectively. c) Schematic reaction of the oxidation of the produced hydrogen peroxide at the Pt working electrode for amperometric detection inside the electrochemical cell.

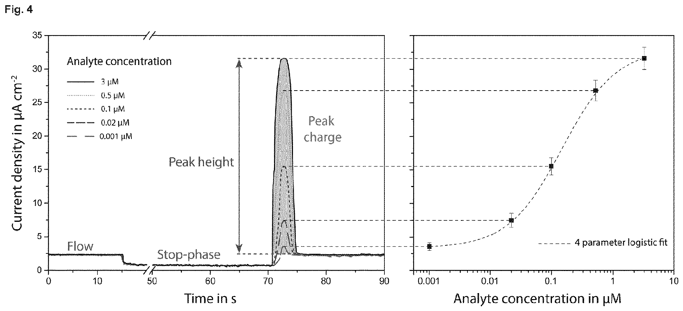

**Figure 4****:** Electrochemical signal readout of an enzyme-linked assay, using the stop-flow technique. a) A flow of 40 mM glucose solution at a rate of 20 µl min⁻¹ was applied. During the stop-phase (1, 2 or 5 min), the enzyme's production of H₂O₂ continues. By restarting the flow, the accumulated H₂O₂ is flushed through the electrochemical cell, where the hydrogen peroxide is electrochemically detected, resulting in an on-chip calibration curve b).
**Figure 5** Illustration of different assays for the formation of an analyte-dependent catalyst complex at the site of the immobilization areas. Herein, the different assays are depicted by illustrating ELISA schemes for an antigen detection. The immobilization assays can be distinguished into direct (left) and indirect formats (right). In a direct format the reporter enzymes are linked to an antibody and the enzyme-antibody complex is introduced to absorb the antigen. The indirect format involves an additional binding process of primary and secondary antibodies. Herein, the primary antibodies are introduced and bind to the antigen. In an additional step, secondary antibodies linked to the reporter enzyme are introduced. Both methods can be combined with different capture schemes for the antigens. In sandwich assays the antigens are captured between two layers of antibodies (i.e. capture and detection antibody). In competitive assays labelled and unlabelled antigen compete for the binding site to the biorecognition elements. In direct assays the antigen bind directly to the substrate.

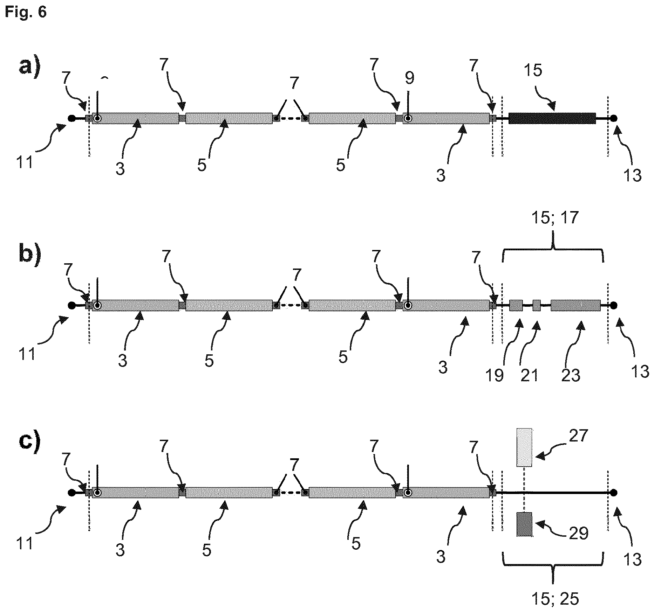

**Figure 6****:** Schematic chip design of a preferred embodiment of a single-channel multianalyte biosensor according to the invention, a) As depicted, the single microfluidic channel comprises N immobilization areas and N-1 spacer areas that are arranged alternating and are separated by stopping barriers. Individual channel inlets 1 through N allow for a deposition of biorecognition elements at the immobilization areas, wherein the spreading of solutions comprising the biorecognition elements is restricted by the stopping barriers. Thus, the biorecognition elements are immobilized specifically within the immobilization areas and not in the intermediate spacer areas. Multiplexing of the sample can be performed as described herein by introducing the sample comprising the analytes followed by a reporter catalyst that immobilizes in the immobilization areas. The introduction of a substrate solution by means of the stop-flow technique allows for a precise readout of the target molecules. For a detection of the target molecules an electrochemical cell comprising a working electrode (WE), reference electrode (RE) and a counter electrode (CE) may be preferred (b). Alternatively, a detection of the target molecules may be performed by an optical cell comprising a light source and a photocell (c).

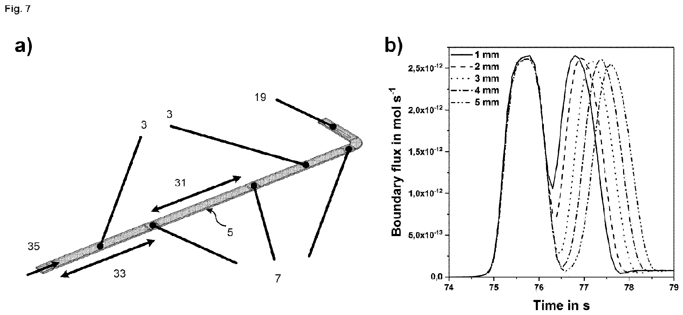

**Figure 7:** a) Illustration of a model of a microfluidic multiplexed sensor with two immobilization areas separated by stopping barriers and a spacer area for a dual analyte detection. b) Results of numerical simulations for said microfluidic multiplexed biosensor. In this case an optimized spacer length for a complete signal separation of the signals stemming from the first and second immobilization area can be reached at a spacer length of 5 mm.

### Examples

The examples disclose how a system for multiplexing analytes in a biosensor can be prepared and used according to preferred embodiments of the invention. In this regard reference is also made to

### Example 1: Fabrication of a microfluidic biosensor in dry film photoresist technology

The following protocol was used to prepare a microfluidic biosensor in dry film photoresist technology.

| 1.1. | Preparation of the polyimide wafers |
|---|---|
| 1.1.1. | Cut a polyimide (PI) substrate to 6 inch round wafers. |
| 1.1.2. | Put the PI wafer into an oven at 120 °C for roughly 1 hr for a dehydration bake. |

| 1.2. | First photolithography step for a lift-off process |
|---|---|
| 1.2.1. | Program the spin coater to a 30 sec spinning time at 3,000 rpm with an acceleration of 2,000 rpm sec⁻¹. |
| 1.2.2. | Place the PI wafer on the spin coater and fix it, applying a vacuum. Dispense 2 ml of a resist (MA-N 1420, micro resist technology), enabling a lift-off process, before starting the spin coater program. |
| 1.2.3. | Soft bake the PI wafer on a hot plate for 2 min at 100 °C. |
| 1.2.4. | Adjust the mask for patterning the electrodes on the PI wafer and expose it to 400 mJ cm⁻² UV light (365 nm). |
| 1.2.5. | Develop the resist on an orbital shaker for 1 min, using the appropriate developer. |
| 1.2.6. | Rinse and dry the PI wafer as described in 1.1.3. |

| 1.3. | Deposition of platinum for the formation the electrodes |
|---|---|
| 1.3.1. | Use a standard physical vapour deposition process to deposit 200 nm of platinum onto the wafer. |
| 1.3.2. | Remove the lift-off resist with its remover on an orbital shaker until all excess platinum is removed. |
| 1.3.3. | Rinse and dry the PI wafer as described in 1.1.3. |

| 1.4. | Second photolithography step, forming the insulation layer |
|---|---|
| 1.4.1. | Put the PI wafer in an oven, preheated to 120 °C, in order to dehydrate them. |
| 1.4.2. | Program the first step of a spin coater to 5 sec spinning time at 500 rpm with a second step, lasting 30 sec at 4,000 rpm with an acceleration of 700 rpm sec⁻¹. |
| 1.4.3. | Place the PI wafer on the spin coater and fix it, applying a vacuum. Dispense 6 ml of an appropriate epoxy-based photoresist (SU-8 3005, MicroChem Corp.), before starting the spin coater program. |
| 1.4.4. | Soft bake the coated wafer for 3 min at 95 °C on a hot plate. |
| 1.4.5. | Adjust the mask for the insulation layer on the wafer, using the respective alignment marks. Expose the wafer to 100 mJ cm⁻² UV light (365 nm). |
| 1.4.6. | For a post exposure bake, place the wafer on the preheated hot plate for 2 min at 95 °C. |
| 1.4.7. | Develop the resist in a suitable developer (e.g., 1-methoxy-2-propyl-acetat in the case of SU-8 for 2 min) on an orbital shaker. |
| 1.4.8. | Rinse the PI wafer first with isopropanol and then rinse and dry it as described in 1.1.3. |
| 1.4.9. | Hard bake the photoresist in an oven for 3 hr at 150 °C. |

| 1.5. | Plasma assisted cleaning of the electrodes |
|---|---|
| 1.5.1. | In order to remove photoresist residues, use an oxygen plasma with a standard plasma unit. Start the cleaning program, using 300 W low frequency power with 100 % of O₂ flow and a total time of 3 min. |
| 1.5.2. | Place the PI wafer into the plasma chamber and fixate the wafer on the grounded plate with glass lids. |

| 1.6. | Silver and silver chloride deposition, creating an on-chip reference electrode |
|---|---|
| 1.6.1. | Passivate the platinum contact pads of the wafer with UV sensitive adhesive tape. Cut therefore the foil in to 5 mm wide and 11 cm long stripes and attach them on the wafer, protecting the parts, not to be deposited with silver. |
| 1.6.2. | For the silver deposition, put a sonic bath into a fume cupboard and insert a container of silver electrolyte solution into the bath. |
| 1.6.3. | Set the sonic bath to room temperature and the power to 10 %. |
| 1.6.4. | Connect the bulk contact of the reference electrodes to a constant current source. Connect the counter electrode, a silver wire, immersed in the silver electrolyte solution, to the current source. |
| 1.6.5. | Set the current source to DC and to a current density of -4.5 mA cm⁻², resulting in a deposition rate of approximately 0.3 µm min⁻¹. Start the sonic bath, and let the current source run for 10 min. |
| 1.6.6. | Rinse the wafer with DI-water |
| 1.6.7. | For the chlorination of the reference electrode, insert a vessel of 0.1 M potassium chloride (KCI) solution into the sonic bath. Connect the wafer and a platinum electrode, connecting the KCI solution, with the constant current source. |
| 1.6.8. | Set the current source to DC and to a current density of +0.6 mA cm⁻², resulting in a deposition rate of approximately 0.075 µm min⁻¹. Start the sonic bath, and let the current source run for 7.5 min. |
| 1.6.9. | Rinse and dry the PI wafer as described in 1.1.3. |
| 1.6.10. | Remove the UV sensitive tape after an UV exposure of 3 min. |
| 1.6.11. | Rinse and dry the PI wafer again as described in 1.1.3. |

| 1.7. | Third photolithography step, using dry film photoresists, creating the microfluidic channels |
|---|---|
| 1.7.1. | Cut the needed dry resist film (DFR) layers (Pyralux^{®} PC 1025, DuPont), to a similar size of the wafer. Fix the DFR onto the respective mask and illuminate the resist with 250 mJ cm⁻² UV light (365 nm). |
| 1.7.2. | Remove the protective foil of the photoresist and develop the resist for roughly 2 min (depending on the structures) in a 1 % sodium carbonate (Na₂CO₃) solution, using a standard sonic bath, preheated to 42 °C and a sonic power of 100 %. |
| 1.7.3. | To stop immediately the reaction, shake the resist for 1 min in a 1 % HCl bath on an orbital shaker. |
| 1.7.4. | Rinse and dry each DFR layer as described in 1.1.3. |
| 1.7.5. | In total, four DFR layers need to be processed the way mentioned above: one channel layer, one cover layer, and two backside layers. |

| 1.8. | Lamination of the developed DFR layers onto the polyimide substrate |
|---|---|
| 1.8.1. | Adjust the channel DFR layer on the PI wafer, using the respective alignment marks. When aligned, fix the DFR layer with adhesive tape. |
| 1.8.2. | For the lamination, use a standard hot roll laminator and preheat the top roll to 100 °C and the bottom roll to 60 °C. The pressure is set to be to 3 bars with a forward speed of 0.3 m min⁻¹. |
| 1.8.3. | Adjust and laminate the both backside layers with the same protocol above. |

| 1.9. | Employing a hydrophobic stopping barrier and sealing of the chip |
|---|---|
| 1.9.1. | Remove the protective layer off the front side DFR channel layer, using an adhesive tape. |
| 1.9.2. | Use a hand dispenser with 0.004" tubes to dispense small drops of dissolved polytetrafluoroethylene (Teflon^{®} AF 1600, DuPont) into the wells of the insulation layer. |
| 1.9.3. | To seal the microfluidic chip, laminate the cover DFR layer onto the channel layer, as described in 1.8. |

| 1.10. | Hard bake of the microfluidic biosensor |
|---|---|
| 1.10.1. | Remove all protective foils of the DFR layers and cut the biosensors into stripes, using an ordinary pair of scissors. |
| 1.10.2. | Cure the chips in an oven at 160 °C for 3 hr. |

The fabrication of the microfluidic biosensor chips is completely realized on wafer-level by standard photolithographic techniques, employing multiple DFR layers. This fabrication strategy relies on the lamination of developed layers of DFRs on a platinum patterned polyimide substrate, forming the microfluidic channels. A short summary depicting the different fabrication steps is given in Fig. 2.

Each biosensor of the example comprises one microfluidic channel, separated by a hydrophobic stopping barrier into two distinct areas; an immobilization section and an electrochemical cell (see Fig. 3) respectively. The electrochemical cell includes an on-chip silver/silver chloride reference electrode, and counter and working platinum electrode. This separation of the immobilization area and the electrochemical readout of the assay, prevents any contamination of the electrodes with biomolecules and therefore inhibits electrode fouling. Furthermore, it enables the precise metering of the immobilization reagents by capillary filling.

By employing DFRs for the fabrication of the sensor, the manufacturing process allows a high-throughput on wafer-level and therefore the costs can be kept down to minimum. The development of all DFR layer are done by using simple and low-cost foil masks, combined with a vacuum exposure unit, instead of using costly chrome masks and mask aligner. In addition, the reduction of the necessary clean room process steps to a minimum cuts the costs for fabrication further more.

In total, the fabrication procedure takes a workload of roughly 10 hr, excluding the baking times and the physical vapour deposition of the platinum.

### Example 2: On-chip assay immobilization procedure

The following protocol was used to immobilize biorecognition elements in the immobilization area of a microfluidic channel.

| 2.1. | Adsorption of avidin in the immobilization area of the channel |
|---|---|
| 2.1.1. | Prepare a 100 µg ml⁻¹ avidin solution in 10 mM phosphate buffered saline (PBS) at a pH of 7.4. |
| 2.1.2. | Dispense 2 µl of the avidin solution onto the inlet of the biosensor. The channel is filled by capillary forces, until the fluid reaches the hydrophobic stopping barrier. |
| 2.1.3. | To ensure that the fluidic keeps stopping at the barrier during the whole incubation time, dispense 2 µl of DI-water on the outlet of the chip. |
| 2.1.4. | Incubate the chip at 25 °C for 1 hr in a closed container. |
| 2.1.5. | Remove excess reagents through the inlet of the chip, by applying a vacuum. Wash the channel with 50 µl of wash buffer (PBS with 0.05 % Tween 20), dispensed on the outlet while the vacuum is applied. |
| 2.1.6. | Dry the channel, while the vacuum is applied, for 30 sec. |

| 2.2. | Blocking of the channel surface to inhibit unspecific binding |
|---|---|
| 2.2.1. | Use 1 % of bovine serum albumin (BSA) solution in 10 mM PBS at a pH of 7.4. |
| 2.2.2. | Pipette 2 µl of the BSA solution on the inlet and 2 µl of DI-water on the outlet of the channel as described in 2.1.2 and 2.1.3. |
| 2.2.3. | Incubate the chip at 25 °C for 1 hr in a closed container. |
| 2.2.4. | Remove excess reagents and dry the channel as described in 2.1.5 and 2.1.6. |

| 2.3. | Incubation of DNA oligonucleotides, labeled with biotin and 6-FAM |
|---|---|
| 2.3.1. | Prepare different concentration (0.001 µM - 5 µM) of the DNA solution in 10 mM PBS at a pH of 7.4. |
| 2.3.2. | Dispense 2 µl of one concentration of the DNA solution on the inlet and 2 µl of DI-water on the outlet as described in 2.1.2 and 2.1.3. |
| 2.3.3. | Incubate the chip at 25 °C for 15 min in a closed container. |
| 2.3.4. | Repeat the steps 2.3.2 and 2.3.3 for the other DNA concentrations, using additional biosensor chips or different immobilization areas in case of biosensor chips with multiple immobilization areas according to preferred embodiments of the invention. |
| 2.3.5. | Remove excess reagents and dry the channel as described in 2.1.5 and 2.1.6. |

| 2.4. | Immobilization of GOx labeled 6-FAM antibodies. |
|---|---|
| 2.4.1. | Formulate a concentration of 5 µg ml⁻¹ in 10 mM PBS at a pH of 7.4. |
| 2.4.2. | Introduce 2 µl of the antibody solution on the inlet and 2 µl of DI-water on the outlet of the channel as described in 2.1.2 and 2.1.3. |
| 2.4.3. | Incubate the labelled antibodies at 25 °C for 15 min in a closed container. |
| 2.4.4. | Remove excess reagents as described in 2.1.5. |

The on-chip assay incubation is preferably realized by capillary forces only. By pipetting drops of different reagents onto the inlet of the microfluidic channel, the fluids are driven through the channel by capillary forces until they reach the hydrophobic stopping barrier. Under steady-state conditions, the reagents are then incubated for a distinct time. This passive system enables the capillary filling of the channel without the need of any external instrumentation, like a syringe pump, and allows the precise metering of the reagents, as the fluid automatically stops at the stopping barrier. Besides that, it allows to keep the workflow of a conventional ELISA, and also works with high viscosity liquids like serum or blood.

For a proof-of-principle experiment, the chip operation is demonstrated by a simple test assay employing avidin-biotin interaction, as shown in Fig. 3. The on-chip assay incubation starts with the adsorption of the avidin to the capillary surface for one hour, followed by a blocking step with bovine serum albumin (BSA) for another hour. Subsequently, 6-FAM/biotin-labeled DNA is incubated in the immobilization capillary for 15 min, where it binds to the avidin molecules. Between each incubation step, any unbound biomolecules are removed by a washing step, applying the wash buffer via the channel outlet, using a custom-made vacuum adapter. In a last step glucose oxidase labeled antifluorescein antibodies are introduced to the channel for 15 min. After that, the biosensor is ready for the electrochemical readout, using a 40 mM glucose solution as substrate.

### Example 3: Amperometric signal detection, using the stop-flow technique

The following protocol was used to for conducting an amperometric signal detection using a glucose solution as a substrate solution for the immobilized reporter enzyme glucose oxidase in a stop-flow assay.

| 3.1. | Preparation of the substrate solution for the electrochemical detection |
|---|---|
| 3.1.1. | Formulate a 40 mM glucose solution in 0.1 M PBS at a pH of 7.4 in ultra-pure water. |
| 3.1.2. | For the preliminary treatment, prepare a 0.1 M PBS solution at a pH of 7.4 in ultra-pure water. |

| 3.2. | Preliminary treatment of the working electrodes |
|---|---|
| 3.2.1. | By using the custom-made chip holder, contact the biosensor electrically to the potentiostat. Realize the fluidic contact of the microfluidic channel to a syringe pump and to the reagent reservoir by using the custom-made adapter. |
| 3.2.2. | Start the syringe pump with 0.1 M PBS at a flow rate of 20 µl min⁻¹. |
| 3.2.3. | Apply at the working electrode an alternating voltage of 0.8 and -0.05 V vs. the on-chip reference electrode for 5 sec each for 30 cycles. |
| 3.2.4. | Following this, oxidize the working electrode by applying a voltage of 0.8 V for 60 sec. |
| 3.2.5. | To start with the assay signal readout, wait until the measured current signal stabilizes. |

| 3.3. | Assay readout, using the stop-flow technique |
|---|---|
| 3.3.1. | Stop the syringe pump and switch the reagent from 0.1 M PBS to the 40 mM glucose solution and restart the pump at 20 µl min⁻¹. |
| 3.3.2. | Wait until the substrate reaches the electrochemical measurement cell and the measured signal stabilizes. |
| 3.3.3. | Stop the flow for 1, 2 or 5 min and restart the flow again. Observe the resulting |
| | anodic current peak. |
| 3.3.4. | For the analysis of the data, either the current peak, or the charge of the peak can be considered. |

For the signal readout of the immobilized assay, the enzymes product (here, H₂O₂), produced in the presence of its substrate glucose, can be electrochemically detected at the working electrode in the electrochemical cell. To achieve a fast detection along with a signal amplification, the so-called stop-flow technique is used (Dincer et al. 2016, US 7691623 B2). In this method, the flow of the enzyme's substrate is stopped, which leads to an accumulation of the enzyme's product inside the capillary. The amount of produced H₂O₂ depends thereby on the amount of bound glucose oxidase and is therefore dependent on the amount of bound biotinylated DNA. By restarting the flow, the enhanced H₂O₂ concentration is subsequently flushed through the electrochemical measurement cell, resulting in a current peak signal, as illustrated in Fig. 4.

For data analysis, the stop-flow technique offers two different parameters, the maximum height and the charge (*i*.*e*., the integral) of the peak signal. Both parameters are directly proportional to the stopping time and can therefore be used for analyzing the data. Considering the data evaluation, using the peak height, the signal height depends on the maximum H₂O₂ concentration and thus, on its diffusion coefficient, as well as the applied stop-time. The longer the stopping time is, the higher is the measured signal response. For this reason, the gauged peak height remains constant down to a minimum length of the immobilization capillary. This special feature of the stop-flow technique allows a drastic decrease in the chip dimensions, particularly in the capillary length, while preserving the sensitivity of the sensor.

### Example 4: Assay systems for analyte-specific catalyst immobilization

Examples 1-3 discloses a system and method for a proof-of-principle of a flow-stop technique using a microfluidic biosensor. It is noted that a person skilled in the art is capable of adapting the above disclosed protocols in order to prepare preferred embodiments of a system and method for multiplexing analytes in a single sample.

For instance, in Example 2 DNA oligonucleotides, labeled with biotin and 6-FAM have been immobilized as biomolecules on the immobilization area. When subsequently introducing glucose oxidase (GOx) labeled 6-FAM antibodies as catalysts, these catalysts immobilize in the immobilisation area.

For a measurement of analytes in a sample instead biorecognition elements such as DNA oligonucleotides or antibodies with a specific binding sites for a desired analyte (e.g. for a testosterone antigen) may be immobilized in the immobilization area by similar method steps. When subsequently introducing the sample (e.g. a plasma sample) the testosterone antigen will bind to the immobilized capture biomolecules. If now a reporter enzyme such as GOx conjugated with an antibody specific for the testosterone antigen is introduced into microfluidic channel. The reporter enzymes will specifically immobilize in the immobilization area. The amount of reporter enzymes immobilizing to the immobilization areas thus directly reflects the amount of testosterone antigens in the sample. By introducing a substrate solution (e.g. a glucose solution) into the channel target molecules (e.g. H₂O₂) are produced that proportionally correlate with the amount of immobilized reporter enzymes. The stop-flow technique allows for measurement of the target molecules by initiating the flow and measuring the amperometric signal using an electrochemical cell.

Figure 5 illustrates different assay schemes for an analyte-specific immobilization of reporter enzymes. In schemes illustrated in Fig. 5 the analyte is an antigen and the specific immobilization is based upon antibody-antigen interactions as in the well-known ELISA assays.

The immobilization assays can be distinguished into direct (left) and indirect assay formats (right). In a direct assay the reporter enzymes are linked to an antibody and the enzyme-antibody complex is introduced to absorb the antigen. The indirect assay involves an additional binding process of primary and secondary antibodies. Herein, the primary antibodies are introduced and bind to the antigen. In an additional step, secondary antibodies linked to the reporter enzyme are introduced. Since multiple secondary antibodies may bind to the primary antibody, indirect assays may increase the sensitivity.

Both methods can be combined with different capture schemes for the analytes, e.g. antigens. In sandwich assays the antigens are captured between two layers of antibodies (i.e. capture and detection antibody). To this end the antigen to be measured should at least comprise two antigenic epitopes capable of binding to antibodies, since at least two antibodies act in the sandwich. Either monoclonal or polyclonal antibodies can be used as capture and detection antibodies. Monoclonal antibodies recognize a single epitope that allows fine detection and quantification of small differences in antigen. A polyclonal is often used as the capture antibody to pull down as much of the antigen as possible.

For a multianalyte assay, different analyte-specific capture antibodies are immobilized to the immobilization areas. Subsequently, the sample comprising the analytes is introduced and the analytes immobilize in the immobilization areas. Next reporter enzymes linked to antigen-specific detection antibody (direct assay) or secondary antigen-specific detection antibodies (indirect assay) are introduced. Herein, either the detection antibodies are chosen such that they bind to all of the antigens or alternatively, multiple different detection antibodies for each of the antigens are used. To allow for the subsequent stop-flow technique a single type of reporter enzyme should be introduced, however different secondary detection antibodies (indirect assay) or detection-antibodies linked to the reporter enzyme (direct assays) may be advantageously employed.

In competitive assays labelled and unlabelled antigen compete for the binding site to capture biomolecules. For a competitive assay labelled versions of antigens to be measured are thus added to the sample. For instance, for each antigen a known quantity of biotinylated antigens can be added to the samples. As capture biomolecules antigen-specific antibodies may be immobilized in the immobilization areas of the microfluidic channel. After introducing the sample at each immobilization site the unlabelled, i.e. endogenous or native antigens, and the introduced labelled antigens compete for the binding to the immobilized capture antibodies. The higher the number of native antigens in the sample the lower will be the proportional binding of labelled antigens at the respective site. The immobilization of the labelled antigens can be determined by introducing reporter enzymes (direct assay) or secondary antibodies (indirect assay) labelled with a binding site for the antigen label. For instance, in case of biotinylated antigens the enzyme or the secondary antibody may be linked to streptavidin in order to allow for a specific immobilization with the antigen complex at the immobilization area.

A readout of the immobilized reporter enzymes is conducted by providing a substrate solution along with a stop-flow measurement. Because the assay is competitive, enzyme activity, i.e. the production of target molecules, is inversely proportional to the amount of (unlabelled native) antigen in the biological sample.

### Example 5: Chip design for a multiplexed immobilization capillary

A preferred chip design of the single channel multianalyte biosensor according to the invention is depicted for different detection principles in Fig. 6. Here, the microfluidic channel contains N immobilization sites each with an individual inlet, which are arranged alternating with spacer areas. That means between each immobilization area, in which biorecognition elements for the analytes are immobilized, a spacer area is present, in which no immobilization occurs. The signal readout for N analytes is realized only by a single measurement cell, independent of the utilized detection method (e.g. optical, electrochemical or calorimetric). In order to ensure a complete signal differentiation, the application of spacer areas, where no immobilization takes place, is essential. Each channel part, such as the immobilization area or the spacer area, is defined in a preferred embodiment by two stopping barriers, preferably hydrophobic or geometric stopping barriers, so that no cross-talk can arise. The facile and compact chip design, similar to lateral flow assays, along with the use of stop-flow protocols facilitates the multiplex detection of various assays in a single channel with a single detection unit, i.e. measurement cell.

### Example 6: Numerical simulations in order to optimize spacer length with respect to the length of the immobilization areas.

In order to demonstrate the proof-of-principle for a multiplexed sensor design numerical simulations have been performed for a model (see Fig. 7a) wherein two immobilization areas are separated by a spacer area. The numerical simulations were carried out through a finite element method (FEM) with COMSOL Multiphysics 5.1 (COMSOL Inc., Sweden) and allowed for a determination of optimized spacer dimensions for a complete signal separation. In numerical simulations, the capillary length of each immobilization area is 5 mm for the given geometries, which has been specified as the optimum channel length maintaining the full sensor performance by the previously introduced design rules (Dincer et al., 2016). Herein, different spacer lengths are applied to the simulation model ranging from 1 mm up to 5 mm. The simulation results of the 1 min stop-flow measurements are depicted in Fig. 7b for different spacer lengths. Pursuant to these numerical results, the total signal separation between different immobilization areas is accomplished at a spacer length of 5 mm, the same as the immobilization area length. This shows that a facile and compact realization of a single-channel multianalyte biosensor is possible, wherein the channel includes N immobilization areas with only a total channel length of N x 1 cm (spacer length 5 mm, immobilization area 5 mm). Together with employing a single measurement cell i.e. an electrochemical cell (Fig. 6b) or with an optical cell (Fig. 6c) this allows for low-cost production of miniaturized multiplexing platforms.

### LIST OF REFERENCE CHARACTERS

- 3: immobilization area
- 5: spacer area
- 7: stopping barrier
- 9: individual channel inlet
- 11: common inlet
- 13: channel outlet
- 15: measurement cell
- 17: electrochemical cell
- 19: working electrode
- 21: reference electrode
- 23: counter electrode
- 25: optical cell
- 27: light source
- 29: photo detector
- 31: length of spacer area
- 33: length of immobilization area
- 35: fluid flow

### REFERENCES

Armbrecht L., Dincer, C., Kling, A., Horak, Kieninger J. and Urban G., Self-assembled magnetic bead chains for sensitivity enhancement of microfluidic electrochemical biosensor platforms, Lab Chip, 2015, 15, 4314.
Bange A., Halsall H. B. and Heineman W. R., Microfluidic immunosensor systems. Biosens. Bioelectron., 20, 2488-2503 (2005).
Betteridge D., Anal. Chem, 50, 832A-846A (1978).
Bruch R., Chatelle C., Kling A., Rebmann B., Wirth S., Schumann S., Weber W., Dincer C. and Urban G., Clinical on-site monitoring of ß-lactam antibiotics for a personalized antibiotherapy. Scientific Reports 7, Article number: 3127 (2017).
Bruch R., Kling A., Urban G., Dincer C., Dry Film Photoresist-based Electrochemical Microfluidic Biosensor Platform: Device Fabrication, On-chip Assay Preparation, and System Operation. Jove-j Vis Exp, 127, e56105 (2017).
Chin C. D., Linder V. and Sia S. K., Lab-on-a-chip devices for global health: past studies and future opportunities. Lab Chip, 7, 41-57 (2007).
Dincer C., Kling, A., et al. Designed miniaturization of microfluidic biosensor platforms using the stop-flow technique. The Analyst 141, 6073-6079, (2016).
Dincer C., Bruch R., Kling A., P. Dittrich S., Urban G. A., Multiplexed Point-of-Care Testing - xPOCT, Trends in Biotechnology 35, 728-742 (2017).
Fu Q., Zhu J. and Van Eyk J. E., Comparison of multiplex immunoassay platforms. Clin. Chem., 56, 314-318 (2010).
Grieshaber D., MacKenzie R., Vörös J., and Reimhult E., Electrochemical Biosensors - Sensor Principles and Architectures, Sensors (Basel), 8(3): 1400-1458 (2008).
Horak, J., Dincer, C., Bakirci, H. & Urban, G. A disposable dry film photoresist-based microcapillary immunosensor chip for rapid detection of Epstein-Barr virus infection. Sensors and Actuators, B: Chemical 191, 813-820, (2014a).
Horak, J., Dincer, C., Bakirci, H. & Urban, G. Sensitive, rapid and quantitative detection of substance P in serum samples using an integrated microfluidic immunochip. Biosensors and Bioelectronics 58, 186-192, (2014b).
Horak, J., Dincer, C., Qelibari, E., Bakirci, H. & Urban, G. Polymer-modified microfluidic immunochip for enhanced electrochemical detection of troponin i. Sensors and Actuators, B: Chemical 209, 478-485, (2015).
Immunoassay Handbook: Theory and Applications of Ligand Binding, ELISA and Related Techniques 4th Edition, Ed. David Wild, Elsevier Oxford UK, (2013).
Kling A., Chatelle C., Armbrecht L., Qelibari L., Kieninger J., Dincer C., Weber W., and Urban G., Multianalyte Antibiotic Detection on an Electrochemical Microfluidic Platform. Analytical Chemistry 88 (20), 10036-10043 (2016).
Ko Y.-J., Maeng J.-H., Ahn Y., Hwang S. Y., Cho N.-G. and Lee S.-H., Electrophoresis, 29, 3466-3476 (2008).
Kuswandia B., Nurimana, Huskensb J. and Verboomb W., Optical sensing systems for microfluidic devices: A review, Analytica chimica acta 601: 141-155 (2007).
Leester-Schädel M., T. Lorenz, F. Jürgens, C. Richter, Fabrication of Microfluidic Devices, Microsystems for Pharmatechnology, Chapter 2, pp 23-57 (2016).
Mehrvar, M.; Abdi, M. Recent developments, characteristics, and potential applications of electro- chemical biosensors. Analytical Sciences 20(8), 1113-1126 (2004).
Pfeiffer S.A. and Nagl S., Microfluidic platforms employing integrated fluorescent or luminescent chemical sensors: a review of methods, scope and applications, Methods Appl. Fluoresc., 3: 034003 (2015).
Ruzicka J. and Hansen E. H., Flow injection analysis, John Wiley & Sons, Inc., New York, Second edi., (1988).
Sackmann E. K., Fulton A. L. and Beebe D. J., The present and future role of microfluidics in biomedical research. Nature, 507, 181-189 (2014).
Spindel S. and Sapsford K. E., Evaluation of optical detection platforms for multiplexed detection of proteins and the need for point-of-care biosensors for clinical use. Sensors (Basel)., 14, 22313-41 (2014).
Whitesides G. M., The origins and the future of microfluidics. Nature, 442, 368-373 (2006).
Yager P., Edwards T., Fu E., Helton K., Nelson K., Tam M. R. and Weigl B. H., Microfluidic diagnostic technologies for global public health. Nature, 442, 412-418 (2006).

## Claims

1. Method for measuring multiple analytes from a single sample comprising the steps of
a) providing a system comprising
i. a common inlet (11) configured to receive a fluid stream
ii. a single microfluidic channel without bifurcation or branching
iii. a measurement cell (15)
iv. a channel outlet (13)
wherein the microfluidic channel comprises at least two immobilization areas (3) and at least one spacer area (5) that are arranged alternating, wherein at the site of the immobilization areas (3) biorecognition elements are immobilized that possess a binding affinity for an analyte
b) introducing the sample comprising at least two analytes into the microfluidic channel via the common inlet (11), whereby first analytes immobilize in a first immobilization area and second analytes immobilize in a second immobilization area
c) introducing a fluid comprising a reporter catalyst into the microfluidic channel via the common inlet (11), whereby the reporter catalyst immobilizes in the respective immobilization areas (3) depending on the presence of analytes to form an analyte-dependent catalyst complex
d) introducing a fluid containing a substrate for the reporter catalyst
e) stopping the flow of the fluid stream for a stop interval such that in each immobilization area (3) a catalytic reaction may take place to produce target molecules
f) initiating a flow of the fluid stream such that at the measurement cell (15) the amount of target molecules produced in each immobilization area (3) can be detected as separate peak signals.

2. Method according to any one of the preceding claims
**characterized in that**
the biorecognition elements are selected from a group consisting of nucleic acids, preferably RNA and/or DNA oligonucleotides, antibodies, peptides, proteins, aptamers, molecularly-imprinted polymers or even whole cells.

3. Method according to any one of the preceding claims
**characterized in that**
in the first immobilization area first biorecognition elements are immobilized with a specific binding affinity for first analytes, whereas in the second immobilization area second biorecognition elements are immobilized with a specific binding affinity for second analytes.

4. Method according to any one of the preceding claims
**characterized in that**
the analyte-dependent catalyst complexes are formed by introducing reporter catalysts conjugated with analyte-specific binding partners and/or introducing in an intermediate step analyte-specific binding partners and subsequently introducing reporter catalysts conjugated with a binding partner for said analyte-specific binding partners.

5. Method according to any one of the preceding claims
**characterized in that**
for each native analyte that is to be measured in the sample a labeled analyte is added, wherein the label constitutes a binding partner to allow for the formation of an analyte-dependent catalyst complex.

6. Method according to any one of claims
**characterized in that**
the reporter catalyst is a reporter enzyme preferably selected from a group consisting of glucose oxidase, horseradish peroxidase, alkaline phosphatase, chloramphenicol acetyl tansferase, β-galactosidase and β-glucuronidase.

7. Method according to any one of the preceding claims
**characterized in that**
the measurement cell (15) is an electrochemical cell (17) comprising one or more working electrode(s) (19), and a common counter electrode (23) and optionally a common reference electrode (21) or
the measurement cell (15) is an optical cell (25) comprising a light source (27) and a photo detector (29).

8. Method according to any one of the preceding claims
**characterized in that**
the amount of produced target molecules is determined based upon the height and/or integral of the signal peak.

9. Method according to any one of the preceding claims
**characterized in that**
the microfluidic channel comprises at least 2, preferably at least 4, most preferably at least 8 immobilization areas (3) and a number of spacer areas (5), which are equal to the number of immobilization areas (3) minus 1.

10. Method according to any one of the preceding claims
**characterized in that**
the cross-sectional dimension of the microfluidic channel is in between 10 nm and 10 mm, preferably between 0.2 µm and 2000 µm, most preferably between 20 µm and 1000 µm,
the length of each of the immobilization area (3) is in between 1 mm and 100 mm, preferably between 5 mm and 50 mm, most preferably between 15 mm and 25 mm
and/or
the length of each of the spacer areas (5) is in between 2 mm and 100 mm, preferably between 5 mm and 50 mm, most preferably between 10 mm and 25 mm.

11. Method according to the preceding claim
**characterized in that**
the length of the stop interval is more than 5 sec, preferably in between 5 sec and 30 min, more preferably between 10 sec and 5 min, most preferably between 20 sec and 2 min.

12. Method according to any one of the preceding claims
**characterized in that**
the microfluidic channel comprises individual inlets (9) for each immobilization area (3) and **in that** the provision of the system includes a method step in which for each immobilization area (3) a fluid comprising biorecognition elements is introduced by said individual inlets (9).

13. Method according to any one of the preceding claims
**characterized in that**
the at least two immobilization areas (3) and the at least one spacer area (5) are separated by stopping barriers (7), wherein preferably
the stopping barriers (7) are hydrophobic sections of the microfluidic channel and/or geometric structures configured to impede capillary spreading of a fluid.

14. Method according to the preceding claim
**characterized in that**
the system comprises an additional measurement cell at the at least one spacer area.

15. System for multiplexing a sample comprising
i. a common inlet (13) configured to receive a fluid
ii. a single microfluidic channel without bifurcation or branching
iii. a measurement cell (15)
iv. a channel outlet (13)
v. control means for introducing the fluid into the common inlet (11) and regulating the fluid stream within the microfluidic channel, wherein the control means include flow inducing or controlling means and a processor unit, wherein the processor unit allows for a combined regulation of the flow inducing or controlling means and the measurement cell
**characterized in that**
the microfluidic channel comprises at least two immobilization areas (3) and at least one spacer area (5) that are arranged alternating, wherein at the site of the immobilization areas (3) biorecognition elements are immobilized that possess a binding affinity for an analyte and wherein the control means are configured to allow for a stop-flow measurement for gauging the production of target molecules by analyte-dependent catalyst complexes at the site of the immobilization areas (3) and wherein the control means are configured to
a) introduce the sample comprising at least two analytes into the microfluidic channel via the common inlet (11), whereby first analytes immobilize in a first immobilization area and second analytes immobilize in a second immobilization area
b) introduce a fluid comprising a reporter catalyst into the microfluidic channel via the common inlet (11), whereby the reporter catalyst immobilizes in the respective immobilization areas (3) depending on the presence of analytes to form an analyte-dependent catalyst complex
c) introduce a fluid containing a substrate for the reporter catalyst
d) stop the flow of the fluid stream for a stop interval such that in each immobilization area (3) a catalytic reaction may take place to produce target molecules
e) initiate a flow of the fluid stream such that at the measurement cell (15) the amount of target molecules produced in each immobilization area can be detected as separate peak signals.

## Patentansprüche

1. Verfahren zum Messen mehrerer Analyten aus einer einzelnen Probe, umfassend die Schritte:
a) Bereitstellen eines Systems, umfassend
i. einen gemeinsamen Einlass (11), der dazu konfiguriert ist, einen Fluidstrom aufzunehmen
ii. einen einzelnen Mikrofluidikkanal ohne Abzweigung oder Verzweigung
iii. eine Messzelle (15)
iv. einen Kanalausgang (13)
wobei der Mikrofluidikkanal mindestens zwei Immobilisierungsbereiche (3) und mindestens einen Spacer-Bereich (5) umfasst, die alternierend angeordnet sind, wobei an der Stelle der Immobilisierungsbereiche (3) Bioerkennungselemente immobilisiert sind, die eine Bindungsaffinität für einen Analyten besitzen
b) Einbringen der mindestens zwei Analyten umfassenden Probe in den Mikrofluidikkanal über den gemeinsamen Einlass (11), wobei erste Analyten in einem ersten Immobilisierungsbereich immobilisieren und zweite Analyten in einem zweiten Immobilisierungsbereich immobilisieren
c) Einbringen eines einen Reporterkatalysator umfassenden Fluids in den Mikrofluidikkanal über den gemeinsamen Einlass (11), wobei der Reporterkatalysator in den jeweiligen Immobilisierungsbereichen (3) in Abhängigkeit von der Gegenwart von Analyten unter Bildung eines analytabhängigen Katalysatorkomplexes immobilisiert
d) Einbringen eines ein Substrat für den Reporterkatalysator enthaltenden Fluids
e) Stoppen der Strömung des Fluidstroms für ein Stoppintervall, sodass in jedem Immobilisierungsbereich (3) eine katalytische Reaktion zum Erzeugm von Zielmolekülen stattfinden kann
f) Initiieren einer Strömung des Fluidstroms, sodass an der Messzelle (15) die Menge der in jedem Immobilisierungsbereich (3) erzeugten Zielmoleküle als separate Peak-Signale detektierbar ist.

2. Verfahren nach einem der vorstehenden Ansprüche,
**dadurch gekennzeichnet, dass**
die Bioerkennungselemente aus einer Gruppe bestehend aus Nukleinsäuren, vorzugsweise RNA- und/oder DNA-Oligonukleotiden, Antikörpern, Peptiden, Proteinen, Aptameren, molekular geprägten Polymeren oder auch ganzen Zellen ausgewählt sind.

3. Verfahren nach einem der vorstehenden Ansprüche,
**dadurch gekennzeichnet, dass**
in dem ersten Immobilisierungsbereich erste Bioerkennungselemente mit einer spezifischen Bindungsaffinität für erste Analyten immobilisiert werden, während im zweiten Immobilisierungsbereich zweite Bioerkennungselemente mit einer spezifischen Bindungsaffinität für zweite Analyten immobilisiert werden.

4. Verfahren nach einem der vorstehenden Ansprüche,
**dadurch gekennzeichnet, dass**
die analytabhängigen Katalysatorkomplexe durch Einbringen von mit analytspezifischen Bindungspartnern konjugierten Reporterkatalysatoren und/oder durch Einbringen von analytspezifischen Bindungspartnern in einen Zwischenschritt und anschließendes Einbringen von mit einem Bindungspartner konjugierten Reporterkatalysatoren für diese analytspezifischen Bindungspartner gebildet werden.

5. Verfahren nach einem der vorstehenden Ansprüche,
**dadurch gekennzeichnet, dass**
für jeden in der Probe zu messenden nativen Analyten ein markierter Analyt zugesetzt wird, wobei die Markierung einen Bindungspartner darstellt, um die Bildung eines analyteabhängigen Katalysatorkomplexes zu ermöglichen.

6. Verfahren nach einem der Ansprüche,
**dadurch gekennzeichnet, dass**
der Reporterkatalysator ist ein Reporterenzym, vorzugsweise ausgewählt aus der Gruppe bestehend aus Glukoseoxidase, Meerrettichperoxidase, alkalischer Phosphatase, Chloramphenicol-Acetyltransferase, β-Galaktosidase und β-Glucuronidase.

7. Verfahren nach einem der vorhergehenden Ansprüche,
**dadurch gekennzeichnet, dass**
die Messzelle (15) eine elektrochemische Zelle (17) ist, die eine oder mehrere Arbeitselektrode(n) (19) und eine gemeinsame Gegenelektrode (23) und optional eine gemeinsame Referenzelektrode (21) umfasst oder
die Messzelle (15) eine optische Zelle (25) ist, die eine Lichtquelle (27) und einen Fotodetektor (29) umfasst.

8. Verfahren nach einem der vorstehenden Ansprüche,
**dadurch gekennzeichnet, dass**
die Menge der erzeugten Zielmoleküle basierend auf der Höhe und/oder des Integrals des Signalpeaks bestimmt wird.

9. Verfahren nach einem der vorstehenden Ansprüche,
**dadurch gekennzeichnet, dass**
der Mikrofluidikkanal mindestens 2, vorzugsweise mindestens 4, am meisten bevorzugt mindestens 8 Immobilisierungsbereiche (3) und eine Anzahl von Spacer-Bereichen (5) auf, die gleich der Anzahl der Immobilisierungsbereiche (3) minus 1 sind, umfasst.

10. Verfahren nach einem der vorstehenden Ansprüche,
**dadurch gekennzeichnet, dass**
die Querschnittsabmessung des Mikrofluidikkanals zwischen 10 nm und 10 mm, vorzugsweise zwischen 0,2 µm und 2000 µm, am meisten bevorzugt zwischen 20 µm und 1000 µm liegt,
die Länge des Immobilisierungsbereichs (3) jeweils zwischen 1 mm und 100 mm, vorzugsweise zwischen 5 mm und 50 mm, am meisten bevorzugt zwischen 15 mm und 25 mm liegt und/oder
die Länge der Spacer-Bereiche (5) jeweils zwischen 2 mm und 100 mm, vorzugsweise zwischen 5 mm und 50 mm, am meisten bevorzugt zwischen 10 mm und 25 mm liegt.

11. Verfahren nach einem der vorhergehenden Ansprüche,
**dadurch gekennzeichnet, dass**
die Länge des Stoppintervalls mehr als 5 s beträgt, vorzugsweise zwischen 5 s und 30 min, mehr bevorzugt zwischen 10 s und 5 min, am meisten bevorzugt zwischen 20 s und 2 min liegt.

12. Verfahren nach einem der vorstehenden Ansprüche,
**dadurch gekennzeichnet, dass**
der Mikrofluidikkanal für jeden Immobilisierungsbereich (3) individuelle Einlässe (9) umfasst und dadurch, dass das Vorsehen des Systems einen Verfahrensschritt beinhaltet, in dem für jeden Immobilisierungsbereich (3) ein Fluid, das Bioerkennungselemente umfasst, durch die individuellen Einlässe (9) eingebracht wird.

13. Verfahren nach einem der vorstehenden Ansprüche,
**dadurch gekennzeichnet, dass**
die mindestens zwei Immobilisierungsbereiche (3) und der mindestens eine Spacer-Bereich (5) durch Stoppbarrieren (7) getrennt, wobei vorzugsweise
die Stoppbarrieren (7) hydrophobe Abschnitte des Mikrofluidikkanals und/oder geometrische Strukturen sind, die dazu konfiguriert sind, eine kapillare Ausbreitung eines Fluids zu verhindern.

14. Verfahren nach einem der vorhergehenden Ansprüche,
**dadurch gekennzeichnet, dass**
das System eine zusätzliche Messzelle an dem mindestens einen Spacer-Bereich umfasst.

15. System zum Multiplexen einer Probe, umfassend
i. einen gemeinsamen Einlass (13), der dazu konfiguriert ist, einen Fluidstrom aufzunehmen
ii. einen einzelnen Mikrofluidikkanal ohne Abzweigung oder Verzweigung
iii. eine Messzelle (15)
iv. einen Kanalausgang (13)
v. Steuermittel zum Einführen des Fluids in den gemeinsamen Einlass (11) und Regulieren des Fluidstroms innerhalb des Mikrofluidikkanals, wobei die Steuermittel strömungsinduzierende oder steuernde Mittel und eine Prozessoreinheit beinhalten, wobei die Prozessoreinheit eine kombinierte Regulierung der strominduzierenden oder steuernden Mittel und der Messzelle ermöglicht,
**dadurch gekennzeichnet, dass**
der Mikrofluidikkanal mindestens zwei Immobilisierungsbereiche (3) und mindestens einen Spacer-Bereich (5) umfasst, die alternierend angeordnet sind, wobei an der Stelle der Immobilisierungsbereiche (3) Bioerkennungselemente immobilisiert sind, die eine Bindungsaffinität für einen Analyten besitzen, und wobei die Steuermittel dazu konfiguriert sind, eine Stopp-Flow-Messung zum Abschätzen der Produktion von Zielmolekülen durch analytabhängige Katalysatorkomplexe an der Stelle der Immobilisierungsbereiche (3) zu ermöglichen, und wobei die Steuermittel zu Folgendem konfiguriert sind:
a) Einbringen der mindestens zwei Analyten umfassenden Probe in den Mikrofluidikkanal über den gemeinsamen Einlass (11), wobei erste Analyten in einem ersten Immobilisierungsbereich immobilisieren und zweite Analyten in einem zweiten Immobilisierungsbereich immobilisieren
b) Einbringen eines einen Reporterkatalysator umfassenden Fluids in den Mikrofluidikkanal über den gemeinsamen Einlass (11), wobei der Reporterkatalysator in den jeweiligen Immobilisierungsbereichen (3) in Abhängigkeit von der Gegenwart von Analyten unter Bildung eines analytabhängigen Katalysatorkomplexes immobilisiert
c) Einbringen eines ein Substrat für den Reporterkatalysator enthaltenden Fluids
d) Stoppen der Strömung des Fluidstroms für ein Stoppintervall, sodass in jedem Immobilisierungsbereich (3) eine katalytische Reaktion zum Erzeugm von Zielmolekülen stattfinden kann
e) Initiieren einer Strömung des Fluidstroms, sodass an der Messzelle (15) die Menge der in jedem Immobilisierungsbereich (3) erzeugten Zielmoleküle als separate Peak-Signale detektierbar ist.

## Revendications

1. Procédé de mesure de plusieurs analytes à partir d'un seul échantillon comprenant les étapes de
a) fourniture d'un système comprenant
i. une entrée commune (11) configurée pour recevoir un courant de fluide
ii. un seul canal microfluidique sans bifurcation ni ramification
iii. une cellule de mesure (15)
iv. une sortie de canal (13)
dans lequel le canal microfluidique comprend au moins deux zones d'immobilisation (3) et au moins une zone d'espacement (5) qui sont disposées en alternance, dans lequel au niveau du site des zones d'immobilisation (3), des éléments de bioreconnaissance sont immobilisés qui possèdent une affinité de liaison pour un analyte
b) introduction de l'échantillon comprenant au moins deux analytes dans le canal microfluidique via l'entrée commune (11), moyennant quoi les premiers analytes s'immobilisent dans une première zone d'immobilisation et les seconds analytes s'immobilisent dans une seconde zone d'immobilisation
c) introduction d'un fluide comprenant un catalyseur rapporteur dans le canal microfluidique via l'entrée commune (11), moyennant quoi le catalyseur rapporteur s'immobilise dans les zones d'immobilisation respectives (3) en fonction de la présence d'analytes pour former un complexe de catalyseur dépendant de l'analyte
d) introduction d'un fluide contenant un substrat pour le catalyseur rapporteur
e) arrêt du flux de courant de fluide pendant un intervalle d'arrêt de telle sorte que, dans chaque zone d'immobilisation (3), une réaction catalytique puisse avoir lieu pour produire des molécules cibles
f) initiation d'un flux de courant de fluide de telle sorte qu'au niveau de la cellule de mesure (15), la quantité de molécules cibles produites dans chaque zone d'immobilisation (3) puisse être détectée sous forme de signaux de crête séparés.

2. Procédé selon l'une quelconque des revendications précédentes **caractérisé en ce que**
les éléments de bioreconnaissance sont choisis dans un groupe constitué d'acides nucléiques, de préférence d'oligonucléotides d'ARN et/ou d'ADN, d'anticorps, de peptides, de protéines, d'aptamères, de polymères à empreinte moléculaire ou même de cellules entières.

3. Procédé selon l'une quelconque des revendications précédentes,
**caractérisé en ce que**
dans la première zone d'immobilisation, des premiers éléments de bioreconnaissance sont immobilisés avec une affinité de liaison spécifique pour les premiers analytes, tandis que dans la seconde zone d'immobilisation, des seconds éléments de bioreconnaissance sont immobilisés avec une affinité de liaison spécifique pour des seconds analytes.

4. Procédé selon l'une quelconque des revendications précédentes **caractérisé en ce que**
les complexes de catalyseur dépendant de l'analyte sont formés par introduction de catalyseurs rapporteurs conjugués à des partenaires de liaison spécifiques de l'analyte et/ou par introduction, dans une étape intermédiaire, de partenaires de liaison spécifiques de l'analyte, puis par introduction de catalyseurs rapporteurs conjugués à un partenaire de liaison pour lesdits partenaires de liaison spécifiques de l'analyte.

5. Procédé selon l'une quelconque des revendications précédentes **caractérisé en ce que**
pour chaque analyte natif qui doit être mesuré dans l'échantillon, un analyte marqué est ajouté, dans lequel le marqueur constitue un partenaire de liaison pour permettre la formation d'un complexe de catalyseur dépendant de l'analyte.

6. Procédé selon l'une quelconque des revendications **caractérisé en ce que**
le catalyseur rapporteur est une enzyme rapporteur choisie de préférence dans un groupe constitué de la glucose oxydase, de la peroxydase de raifort, de la phosphatase alcaline, de la chloramphénicol acétyl tansférase, de la β-galactosidase et de la β-glucuronidase.

7. Procédé selon l'une quelconque des revendications précédentes **caractérisé en ce que**
la cellule de mesure (15) est une cellule électrochimique (17) comprenant une ou plusieurs électrodes de travail (19), et une contre-électrode commune (23) et éventuellement une électrode de référence commune (21) ou
la cellule de mesure (15) est une cellule optique (25) comprenant une source lumineuse (27) et un photodétecteur (29).

8. Procédé selon l'une quelconque des revendications précédentes **caractérisé en ce que**
la quantité de molécules cibles produites est déterminée sur la base de la hauteur et/ou de l'intégrale du pic de signal.

9. Procédé selon l'une quelconque des revendications précédentes **caractérisé en ce que**
le canal microfluidique comprend au moins 2, de préférence au moins 4, de préférence au moins 8 zones d'immobilisation (3) et un nombre de zones d'espacement (5), qui est égal au nombre de zones d'immobilisation (3) moins 1.

10. Procédé selon l'une quelconque des revendications précédentes
**caractérisé en ce que**
la dimension de la section transversale du canal microfluidique est comprise entre 10 nm et 10 mm, de préférence entre 0,2 µm et 2 000 µm, de manière préférée entre toutes entre 20 µm et 1 000 µm,
la longueur de chacune des zones d'immobilisation (3) est comprise entre 1 mm et 100 mm, de préférence entre 5 mm et 50 mm, de manière préférée entre toutes entre 15 mm et 25 mm et/ou
la longueur de chacune des zones d'espacement (5) est comprise entre 2 mm et 100 mm, de préférence entre 5 mm et 50 mm, de manière préférée entre toutes entre 10 mm et 25 mm.

11. Procédé selon la revendication précédente
**caractérisé en ce que**
la durée de l'intervalle d'arrêt est supérieure à 5 secondes, de préférence entre 5 secondes et 30 minutes, plus préférablement entre 10 secondes et 5 minutes, de manière préférée entre toutes entre 20 secondes et 2 minutes.

12. Procédé selon l'une quelconque des revendications précédentes
**caractérisé en ce que**
le canal microfluidique comprend des entrées individuelles (9) pour chaque zone d'immobilisation (3) et **en ce que** la fourniture du système comprend une étape de procédé dans laquelle pour chaque zone d'immobilisation (3), un fluide comprenant des éléments de bioreconnaissance est introduit par lesdites entrées individuelles (9).

13. Procédé selon l'une quelconque des revendications précédentes
**caractérisé en ce que**
les au moins deux zones d'immobilisation (3) et l'au moins une zone d'espacement (5) sont séparées par des barrières d'arrêt (7), dans lequel de préférence
les barrières d'arrêt (7) sont des sections hydrophobes du canal microfluidique et/ou des structures géométriques configurées pour empêcher la propagation capillaire d'un fluide.

14. Procédé selon la revendication précédente
**caractérisé en ce que**
le système comprend une cellule de mesure supplémentaire au niveau de l'au moins une zone d'espacement.

15. Système de multiplexage d'un échantillon comprenant
i. une entrée commune (13) configurée pour recevoir un fluide
ii. un seul canal microfluidique sans bifurcation ni ramification
iii. une cellule de mesure (15)
iv. une sortie de canal (13)
v. un moyen de contrôle pour introduire le fluide dans l'entrée commune (11) et réguler le courant de fluide dans le canal microfluidique, dans lequel le moyen de contrôle comprend un moyen d'induction ou de contrôle du débit et une unité de processeur, dans lequel l'unité de processeur permet une régulation combinée du moyen d'induction ou de contrôle du débit et de la cellule de mesure
**caractérisé en ce que**
le canal microfluidique comprend au moins deux zones d'immobilisation (3) et au moins une zone d'espacement (5) disposées en alternance, dans lequel au niveau du site des zones d'immobilisation (3), des éléments de bioreconnaissance sont immobilisés qui possèdent une affinité de liaison pour un analyte et dans lequel le moyen de contrôle est configuré pour permettre une mesure d'arrêt du flux pour évaluer la production de molécules cibles par des complexes de catalyseur dépendant d'un analyte au niveau du site des zones d'immobilisation (3) et dans lequel le moyen de contrôle est configuré pour
a) introduire l'échantillon comprenant au moins deux analytes dans le canal microfluidique via l'entrée commune (11), moyennant quoi les premiers analytes s'immobilisent dans une première zone d'immobilisation et les seconds analytes s'immobilisent dans une seconde zone d'immobilisation
b) introduire un fluide comprenant un catalyseur rapporteur dans le canal microfluidique via l'entrée commune (11), moyennant quoi le catalyseur rapporteur s'immobilise dans les zones d'immobilisation respectives (3) en fonction de la présence d'analytes pour former un complexe de catalyseur dépendant de l'analyte
c) introduire un fluide contenant un substrat pour le catalyseur rapporteur
d) arrêter le flux de courant de fluide pendant un intervalle d'arrêt de telle sorte que, dans chaque zone d'immobilisation (3), une réaction catalytique puisse avoir lieu pour produire des molécules cibles
e) initier un flux de courant de fluide de telle sorte qu'au niveau de la cellule de mesure (15), la quantité de molécules cibles produites dans chaque zone d'immobilisation puisse être détectée sous forme de signaux de crête séparés.
